# EUROPEAN PATENT APPLICATION

(11) **EP 3 988 546 A1**
(43) Date of publication of application: **27.04.2022**
(21) Application number: 20827805.1
(22) Date of filing: 22.06.2020
(51) Int. Cl.: C07D 401/14, C07D 471/04, A61K 31/506, A61P 35/00, A61P 35/02

(54) **SALTS OF COMPOUND, CRYSTALLINE FORMS THEREOF, PREPARATION METHOD THEREFOR AND APPLICATION THEREOF**

(30) Priority: 21.06.2019 CN 201910542355
(71) Applicant: Gan & Lee Pharmaceuticals Co., Ltd., Beijing 101109 (CN)
(72) Inventor: YIN, Lei, Beijing 101109 (CN); YAO, Zhenglin, Beijing 101109 (CN)
(74) Representative: Ladendorf, Oliver
(86) International application number: PCT/CN2020/097482
(87) International publication number: WO 2020/253875

(57) **Abstract**

The present invention relates to the fumarate, maleate, adipate, and succinate of a compound 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, multiple crystal forms of the salts and preparation method and use thereof.

It has been confirmed that the above salts or crystal forms thereof can better replace 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, so as to overcome its shortcomings in solubility, high risk of food effects, stability and the like, and meanwhile, the above salts or crystal forms thereof have a relatively low hygroscopicity, and high application value.

## Description

### Cross Reference to Related application

This application claims the priority of Chinese patent application No. CN201910542355.6, filed on June 21, 2019, which is incorporated herein by reference in its entirety.

### Technical Field

The present invention relates to the field of medicines, in particular to a series of salts of a CDK4/6 inhibitor, crystal forms of the salts and preparation method and use thereof.

### Background Art

The cell cycle is an important part of cell life activities. In the normal cell growth process, the realization of the cell cycle process depends on the precise and strict regulation of the cell cycle by various levels of regulatory factors. The core of these regulatory factors comprises a cyclin dependent kinase (CDK) and its positive and negative regulators, i.e. cyclin and cyclin dependent kinase inhibitor (CDI). The CDK-Cyclin complex formed by the cyclin dependent kinase and cyclin participates in cell growth, proliferation, dormancy or apoptosis. During the cell cycle, cyclins are continuously expressed and degraded periodically, and respectively bind to the CDK transiently activated by them. Through the activity of CDK, the phosphorylation of different substrates is catalyzed to achieve the promoting and transforming effect on different phases of the cell cycle.

At present, 13 members of the CDK family have been found, namely CDK1 to CDK13; wherein CDK1, CDK2, CDK3, CDK4 and CDK6 are involved in regulating cell proliferation, and CDK7, CDK8, CDK9, CDK11, CDK12 and CDK13 are involved in regulating transcription.

Cyclin is divided into A to L, and different CDKs are connected to different subtypes of Cyclin. Among them, the Cyclin D family (Cyclin D1, D2, D3) begins to express in the G1 phase, and binds and activates CDK4 and CDK6 to form the CDK4/6-Cyclin D complex, so that a series of substrates including retinoblastoma protein (Rb) are phosphorylated. After phosphorylation, Rb releases the proteins that bind to it and are inhibited by it, mainly transcription factors such as E2F, and E2F activates and transcribes some genes necessary for entry into S phase (MA Ke, Research progress in antitumor effect of a CDK4/6 inhibitor, World Notes on Antibiotics, 2013, 34 (5): 197-202). If the balance is broken due to various factors, no matter the signal that promotes cell proliferation is increased or the signal that inhibits cell proliferation is reduced to a certain extent, cell proliferation will be out of control, and tumors will appear. In the study, it has found that about 80% of human tumors have abnormalities in the Cyclin D-CDK4/6-INK4-Rb pathway (1. Malumbres M, Barbacid M., To cycle or not to cycle: a critical decision in cancer [J]. Nature Reviews Cancer, 2001, 1 (3): 222; and 2. Shapiro GI., Cyclin-dependent kinase pathways as targets for cancer treatment [J]. J Clinical Oncology, 2006, 24 (11): 1770). The change of this pathway accelerates the progress of G1 phase, so that the proliferation of tumor cells is accelerated and a survival advantage is obtained. Therefore, intervention to this pathway has become a therapeutic strategy, and CDK4/6 has become one of the potential targets for antitumor treatment.

The advantages of CDK4/6 as a target for antitumor treatment lie in that, (1) most proliferating cells rely on CDK2 or CDK4/6 to proliferate, but inhibitors of CDK4/6 do not exhibit the cytotoxicity of "pan-CDK inhibitors", such as bone marrow suppression and intestinal reactions; and (2) preclinical experiments have shown that if the cell Cyclin D level is increased or p16INK4a is inactivated, the sensitivity of cells to drugs can increase, and because of the above phenomenon in tumor cells relative to normal cells, the drug targeting is increased to a certain extent.

In addition to inhibiting tumor growth, CDK inhibitors are also used for the treatment of other diseases, for example, for the treatment of cardiovascular disorders, including atherosclerosis, restenosis after vascular stent implantation, and other cardiovascular disorders caused by abnormal cell proliferation, for another example, for the treatment of diseases caused by fungi, protozoan parasites (such as Plasmodium falciparum) and DNA and RNA virus infections, including malaria, AIDS and the like. In addition, it has been found in studies that CDK inhibitors can also be used for autoimmune system diseases (such as psoriasis, rheumatoid arthritis, glomerulonephritis, and lupus erythematosus), and for inhibiting the proliferation of inflammatory cells.

The disclosed in Chinese application CN 106810536 is an effective CDK4/6 inhibitor, 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, as represented by formula I:

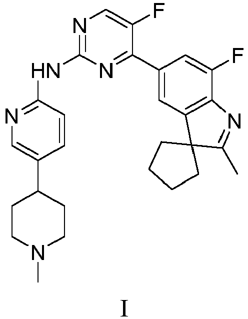

Although this compound is an effective CDK4/6 inhibitor, there are still problems with the application of the compound in pharmaceutical products, for example, 1) the free base (also referred to as free amine) of the compound has a low solubility in water. Even though the optimal crystal form A of the compound (i.e., Comparative Example 1 below) is selected, the solubility thereof in water is only 75 µg/mL, and a relatively high food effect appears in a simulated intestinal fluid, that is, the difference between the solubility in fasted state simulated intestinal fluid (FaSSIF) and the solubility in fed state simulated intestinal fluid (FeSSIF) is large, and the risk of food effect is high. When the compound is applied to patients, it has a high risk of food effect, and poor stability. Under a condition of slightly high temperature or light illumination (e.g. 4500 lux), and conditions of high temperature and high humidity (e.g. 40°C/75% RH), the purity of the compound is reduced, which is unfavorable to the application and storage of the compound. Therefore, it is urgent to propose suitable alternatives to solve such problems.

### Summary of the Invention

The first purpose of the present invention is to provide a salt of a CDK4/6 inhibitor, so as to better replace 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to overcome its shortcomings in solubility, high risk of food effects, stability and the like, and meanwhile, the above salt has a relatively low hygroscopicity.

Specifically, the present invention provides a salt of a compound represented by formula I:

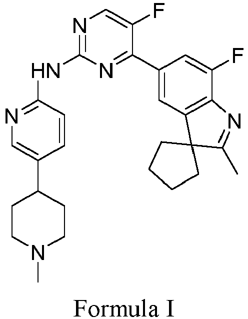

The salt is selected from fumarate, maleate, adipate or succinate, i.e., four salts of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine: fumarate, maleate, adipate and succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

Although 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine has been proved to be an effective CDK4/6 inhibitor, but subsequent studies have shown that there are still problems with the application of this compound in pharmaceutical products. In order to look for suitable alternatives, the inventors studied a large number of salts of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, however, in subsequent further studies, it was found that various salts of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine differ greatly in solubility, food effect, hygroscopicity and stability, and many salts have poorer stability and higher hygroscopicity than the free base. In order to obtain better alternatives, numerous experiments were performed to study a large number of salts, including but not limited to hydrochloride, phosphate, lactate, maleate, fumarate, succinate, malate, adipate, tartrate, hippurate, citrate, glycolate, malonate, benzoate, gentisate, sebacate, 1-hydroxy-2-naphthoate, oxalate, methanesulfonate, ethanedisulfonate, benzenesulfonate, p-toluenesulfonate, and hydrobromide, and a large number of crystal forms of the above-mentioned salts obtained under various reaction conditions, and it was unexpectedly found in the present invention that four specific salts, i.e., fumarate, maleate, succinate, and adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine have one or more of the following excellent characteristics compared to 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine and various other salts and crystal forms thereof:
1) higher solubilities in water and fasted state simulated intestinal fluid (FaSSIF);
2) similar solubilities in fasted state simulated intestinal fluid (FaSSIF) and fed state simulated intestinal fluid (FeSSIF), with a relatively low risk of food effects;
3) superior stability; and
4) a relatively low hygroscopicity comparable to the optimal crystal form A of the free base.

As one of the parallel solutions of the present application, the present invention provides a fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

The fumarate has higher solubilities in water and FaSSIF than the free base, and has similar solubilities in FaSSIF and FeSSIF, that is, a lower risk of food effects. Moreover, the crystal form of the fumarate dispersed in water, FaSSIF and FeSSIF is very stable without any change during the solubility test, while other salts of the free base such as methanesulfonate will change in crystal form. At the same time, unexpectedly, the fumarate has a low hygroscopicity comparable to the free base, and the crystal form is stable without any change after absorption of moisture. Generally, in the field of medicines, when a compound is converted from the form of base into a salt, the salt will have a higher hygroscopicity than the free base. For example, each crystal form of the methanesulfonate and hydrochloride of the free base of the present invention has a strong hygroscopicity, and the crystal form will change after absorption of moisture. Furthermore, the fumarate has a better physical and chemical stability than the free base and other salts under conditions of high humidity and light illumination.

In one embodiment, the ratio (here referring to the molar ratio) of the fumarate ion to the free base in fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine is n:1, wherein n ≥ 0.5, preferably n is 0.5, 1 or 2, more preferably n is 1, for example the mono-fumarate represented by the following formula II:

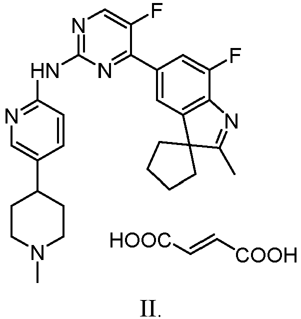

In one embodiment, the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention comprises one or more crystal forms, preferably the fumarate is present in one crystal form or a mixture of multiple crystal forms, particularly preferably the fumarate is present in one crystal form.

In one embodiment, the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention contains solvent molecules (Preferably with a variable content), preferably the salt contains water molecules (Preferably with a variable content).

The term "variable content" in "solvent molecules with a variable content" herein means that the number of solvent molecules in various salts (such as fumarate, adipate, maleate, succinate, etc.) can vary, ideally, for example, the molar ratio of fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to solvent is 1:m, wherein m ≥ 0.5, preferably m is 0.5, 1 , 2, 3, 4 or 5, more preferably m is 0.5 or 1.

Preferably, the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine comprises one or more of the following crystal forms, preferably the fumarate is present in any one of the following crystal forms of fumarate or a mixture of multiple crystal forms of the fumarate, more preferably in one of the following crystal forms, and the X-ray powder diffraction pattern of the crystal form represented by the diffraction angle of 2θ ± 0.2° at least comprises:
(1) characteristic diffraction peaks shown at 7.0°, 13.9°, 18.4°, 21.8° and 24.1°; or
(2) characteristic diffraction peaks shown at 4.5°, 10.5°, 18.3°, 20.6° and 23.9°; or
(3) characteristic diffraction peaks shown at 5.9°, 14.4°, 18.6°, 23.6° and 23.9°;
preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 18.4°, 21.8° and 24.1°;
(2) characteristic diffraction peaks shown at 4.5°, 8.9°, 10.5°, 17.9°, 18.3°, 20.6°, 22.5°, and 23.9°; or
(3) characteristic diffraction peaks shown at 5.9°, 14.4°, 17.6°, 18.6°, 21.6°, 23.6°, 23.9° and 28.4°;
more preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 15.7°, 17.2°, 18.4°, 21.8° and 24.1°; or
(2) characteristic diffraction peaks shown at 4.5°, 8.9°, 10.5°, 17.1°, 17.9°, 18.3°, 20.6°, 22.5°, 23.9° and 27.5°; or
(3) characteristic diffraction peaks shown at 5.9°, 11.7°, 14.4°, 17.6°, 18.6°, 21.6°, 23.6°, 23.9°, 25.0° and 28.4°.

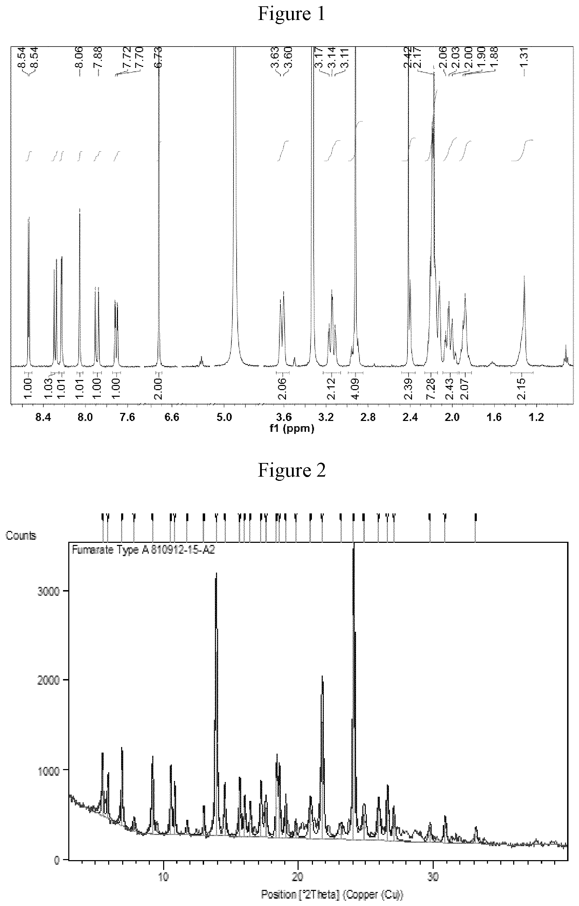

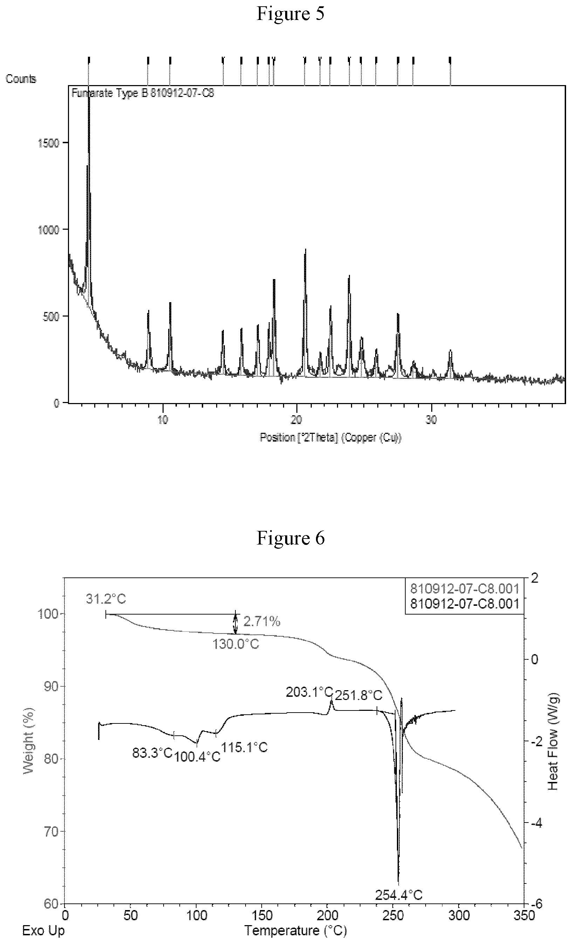

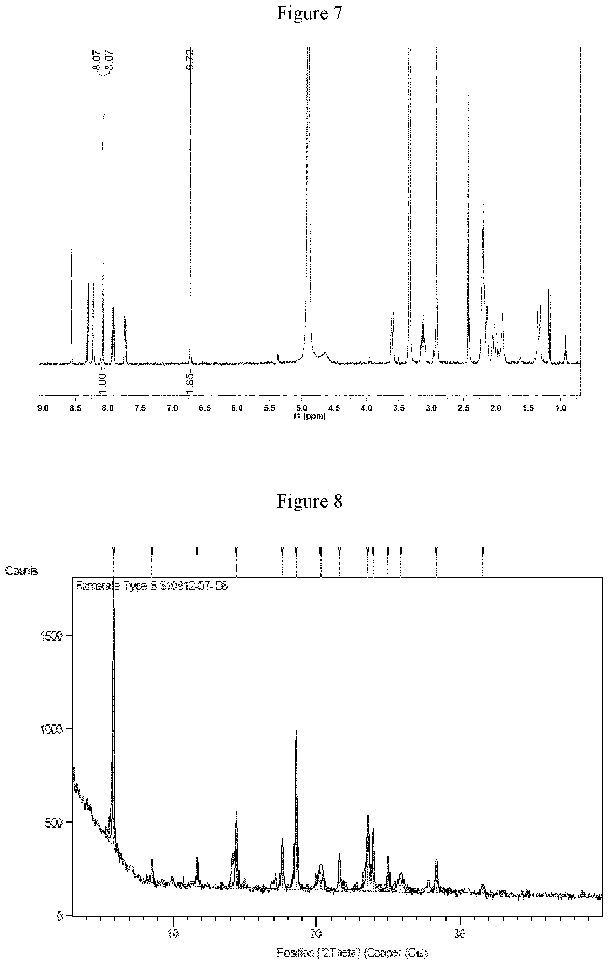

The crystal form of the fumarate particularly preferably has XRPD data and/or a XRPD pattern selected from any one of the following (1) to (3):
(1) XRPD data substantially as shown in Table 1; and/or, a XRPD pattern substantially as shown in FIG. 2;
(2) XRPD data substantially as shown in Table 2; and/or, a XRPD pattern substantially as shown in FIG. 5; and
(3) XRPD data substantially as shown in Table 3; and/or, a XRPD pattern substantially as shown in FIG. 8.

The above-mentioned (1), (2), (3) respectively correspond to XRPD information of crystal form A (hereinafter referred to as fumarate crystal form A), crystal form B (hereinafter referred to as fumarate crystal form B) and crystal form C (hereinafter referred to as fumarate crystal form C) of fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

The above-mentioned crystal forms can be distinguished by X-ray powder diffraction (XRPD), differential scanning calorimetry (DSC), thermogravimetric analysis (TGA), or nuclear magnetic resonance method.

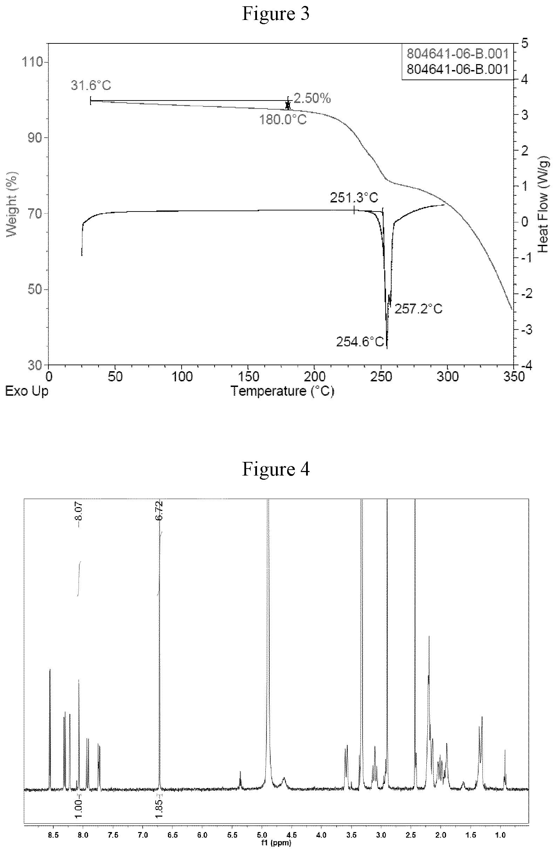

With regard to fumarate crystal form A:
further, the DSC curve of the above-mentioned fumarate crystal form A shows an endothermic peak at 254.6°C;
further, the above-mentioned fumarate crystal form A has a weight loss of 2.50% when heated from room temperature to 180°C;
preferably, the above-mentioned fumarate crystal form A has DSC and TGA curves substantially as shown in FIG. 3.

The fumarate crystal form A described in the present invention has relatively high crystallinity and relatively high melting point, higher solubilities in water and FaSSIF than the free base, and similar solubilities in FaSSIF and FeSSIF, that is, a lower risk of food effects. Moreover, unexpectedly, the fumarate crystal form A has a low hygroscopicity comparable to the free base, and the crystal form is stable without any change after absorption of moisture. At the same time, it has a better physical and chemical stability than the free base under the conditions of high humidity and light illumination.

With regard to fumarate crystal form B:
further, the DSC curve of the above fumarate crystal form B shows an endothermic peak at 254.4°C;
further, the above-mentioned fumarate crystal form B has a weight loss of 2.71% when heated from room temperature to 130°C.

Preferably, the above-mentioned fumarate crystal form B has DSC and TGA curves substantially as shown in FIG. 6.

The fumarate crystal form B described in the present invention has relatively high crystallinity and relatively high melting point, higher solubilities in water and FaSSIF than the free base, and similar solubility in FaSSIF and FeSSIF, that is, a lower risk of food effects. Moreover, unexpectedly, the fumarate crystal form B has a low hygroscopicity comparable to the free base, and the crystal form is stable without any change after absorption of moisture. At the same time, the fumarate crystal form B has a better physical and chemical stability than the free base under the conditions of high humidity and light illumination.

With regard to fumarate crystal form C:
further, the DSC curve of the above-mentioned fumarate crystal form C shows an endothermic peak at 240.2°C;
further, the above-mentioned fumarate crystal form C has a weight loss of 1.95% when heated from room temperature to 130°C.

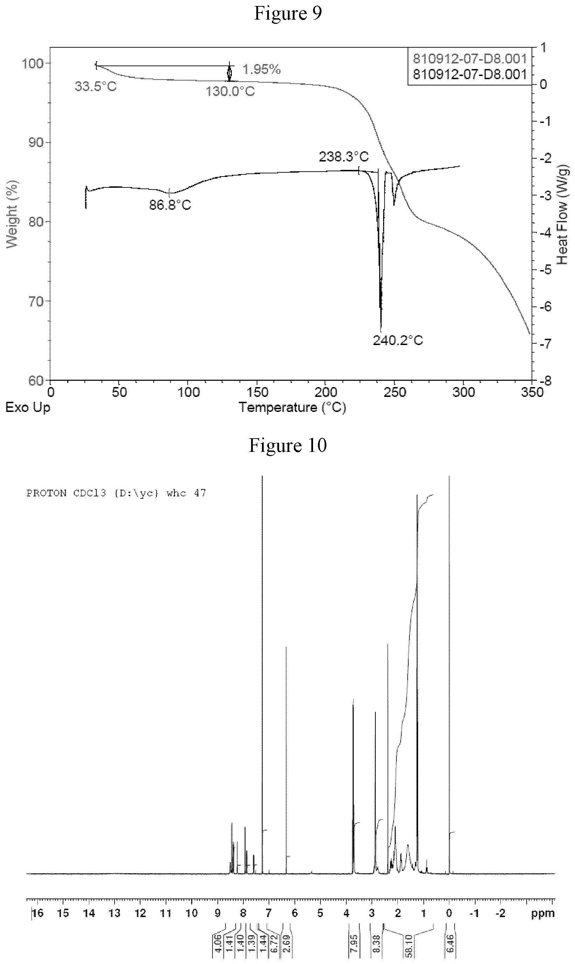

Preferably, the above-mentioned fumarate crystal form C has DSC and TGA curves substantially as shown in FIG. 9.

The fumarate crystal form C described in the present invention has relatively high crystallinity and relatively high melting point, relatively high solubility in FeSSIF. Moreover, unexpectedly, the fumarate crystal form C has a low hygroscopicity comparable to the free base, and the crystal form is stable without any change after absorption of moisture. At the same time, it has a better physical and chemical stability than the free base under high humidity and light illumination conditions.

As the second parallel solution of the present application, the present invention provides a maleate of a CDK4/6 inhibitor, i.e., the maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

The maleate has higher solubilities in water and FaSSIF than the free base, and similar solubilities in FaSSIF and FeSSIF, that is, a lower risk of food effects. Moreover, unexpectedly, the maleate has a low hygroscopicity comparable to the free base, and the crystal form is stable without any change after absorption of moisture. At the same time, the maleate has better physical and chemical stability under the conditions of high humidity and light illumination.

In one embodiment, the ratio of the maleate ion to the free base in maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine is n:1, wherein n ≥ 0.5, preferably n is 0.5, 1 or 2, more preferably n is 1, for example the mono-maleate represented by the following formula III.

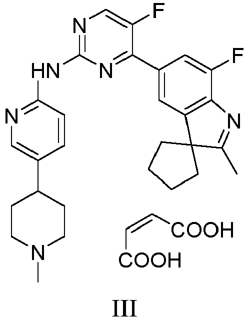

In one embodiment, the maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention comprises one crystal form or multiple crystal forms, preferably the maleate is present in one crystal form or a mixture of multiple crystal forms, particularly preferably the maleate is present in one crystal form.

In one embodiment, the maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention contains solvent molecules (Preferably with a variable content), preferably the salt contains water molecules (Preferably with a variable content).

Preferably, the maleate comprises one or more of the following crystal forms of maleate, preferably the maleate is present in any one of the following crystal forms of maleate or a mixture of multiple crystal forms of the maleate, particularly preferably any one of the following crystal forms of maleate:
The X-ray powder diffraction pattern of the crystal form represented by the diffraction angle of 2θ ± 0.2° at least comprises:
(1) characteristic diffraction peaks shown at 7.8°, 9.0°, 15.7°, 18.4° and 22.7°; or
(2) characteristic diffraction peaks shown at 5.7°, 13.7°, 17.9°, 18.9° and 23.6°.

Preferably, the X-ray powder diffraction pattern of the crystal form of the maleate represented by the diffraction angle of 2θ ± 0.2° at least comprises:
(1) characteristic diffraction peaks shown at 7.3°, 7.8°, 9.0°, 15.7°, 17.9°, 18.4°, 20.7° and 22.7°; or
(2) characteristic diffraction peaks shown at 5.7°, 13.7°, 17.6°, 17.9°, 18.9°, 21.9°, 23.6° and 24.8°.

More preferably, the X-ray powder diffraction pattern of the crystal form of the maleate represented by the diffraction angle of 2θ ± 0.2° at least comprises:
(1) characteristic diffraction peaks shown at 7.3°, 7.8°, 9.0°, 13.6°, 15.7°, 17.9°, 18.4°, 20.7°, 22.7°, and 24.1°; or
(2) characteristic diffraction peaks shown at 5.7°, 13.7°, 14.1°, 17.2°, 17.6°, 17.9°, 18.9°, 21.9°, 23.6° and 24.8°.

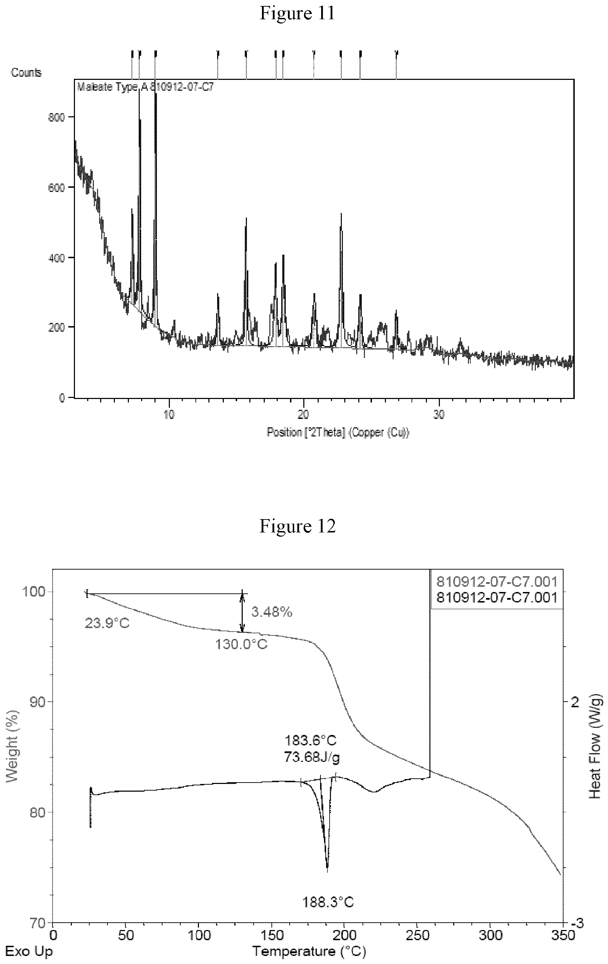

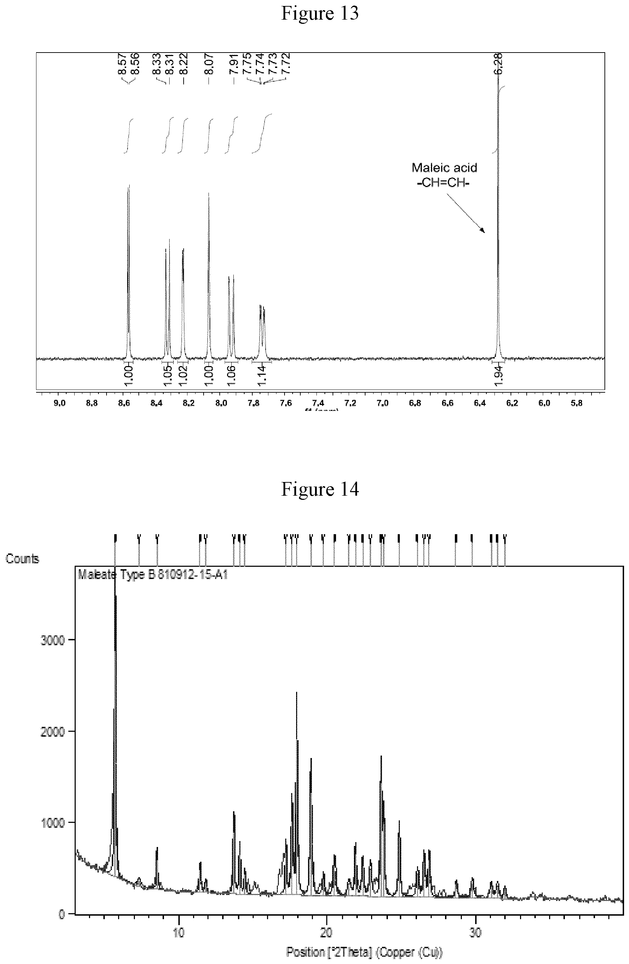

Particularly preferably, the crystal form of the maleate has XRPD data and/or pattern selected from any one of the following (1) to (2):
(1) XRPD data substantially as shown in Table 4; and/or XRPD pattern substantially as shown in FIG. 11; and
(2) XRPD data substantially as shown in Table 5; and/or XRPD pattern substantially as shown in FIG. 14.

The above-mentioned (1) and (2) respectively correspond to XRPD information of crystal form A (hereinafter referred to maleate crystal form A), and crystal form B (hereinafter referred to maleate crystal form B) of maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

The above-mentioned crystal forms can be distinguished by X-ray powder diffraction (XRPD), differential scanning calorimetry (DSC), or nuclear magnetic resonance method.

With regard to maleate crystal form A:
Further, the DSC curve of the above maleate crystal form A shows an endothermic peak at 188.3°C;
Further, the above maleate crystal form A has a weight loss of 3.48% when heated from room temperature to 130°C.
Preferably, the above maleate crystal form A has DSC and TGA curves substantially as shown in FIG. 12.

The maleate crystal form A described in the present invention has relatively high crystallinity and relatively high melting point, higher solubilities in water and FaSSIF than the free base, and similar solubility in FaSSIF and FeSSIF, that is, a lower risk of food effects. Moreover, unexpectedly, the maleate crystal form A has a low hygroscopicity comparable to the free base, and the crystal form is stable without any change after absorption of moisture. At the same time, the maleate crystal form A has a better physical and chemical stability under the conditions of high humidity and light illumination.

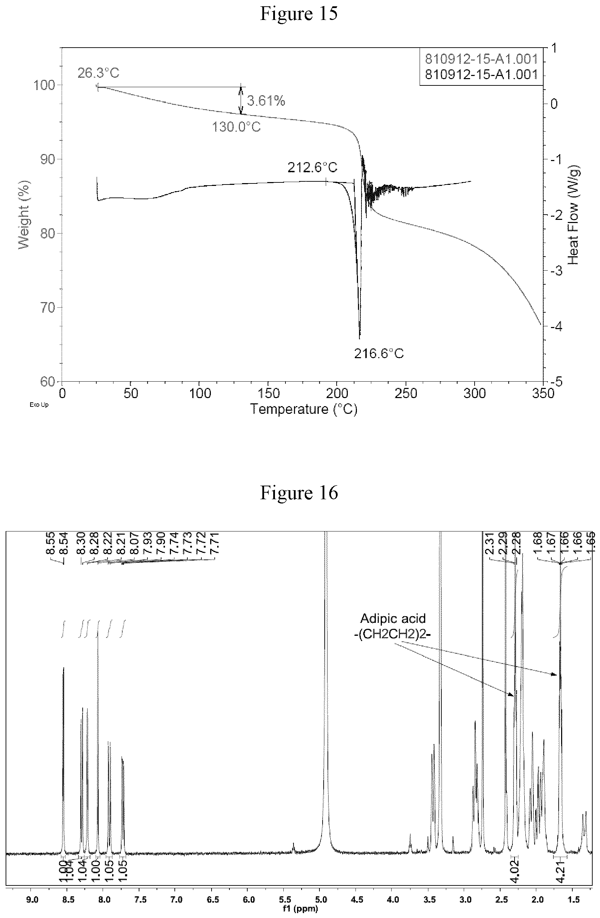

With regard to maleate crystal form B:
Further, the DSC curve of the above maleate crystal form B shows an endothermic peak at 216.6°C;
Further, the above maleate crystal form B has a weight loss of 3.61% when heated from room temperature to 130°C.
Preferably, the above maleate crystal form B has DSC and TGA curves substantially as shown in FIG. 15.

The maleate crystal form B described in the present invention has relatively high crystallinity and relatively high melting point, higher solubilities in water and FaSSIF than the free base, and similar solubility in FaSSIF and FeSSIF, that is, a lower risk of food effects. Moreover, unexpectedly, the maleate crystal form B has a low hygroscopicity comparable to the free base, and the crystal form is stable without any change after absorption of moisture. At the same time, the maleate crystal form B has a better physical and chemical stability under the conditions of high humidity and light illumination.

As the third parallel solution of the present application, the present invention provides an adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine. The adipate has higher solubilities in water and FaSSIF than the free base, and similar solubilities in FaSSIF and FeSSIF, that is, a lower risk of food effects. Moreover, unexpectedly, the adipate has a low hygroscopicity comparable to free base, and the crystal form is stable without any change after absorption of moisture. At the same time, the adipate has a better physical and chemical stability under the conditions of high humidity and light illumination.

In one embodiment, the ratio of the adipate ion to the free base in adipate of 5-fluoro-4-(7'-fluoro-2'-methylspiro[cyclopentane-1,3-indol]-5'-yl)-N-(5-(1-methylpiperidi n-4-yl)pyridin-2-yl)pyrimidine-2-amine of the present invention is n:1, wherein n ≥ 0.5, preferably n is 0.5, 1 or 2, more preferably n is 1, for example the monoadipate represented by the following formula IV.

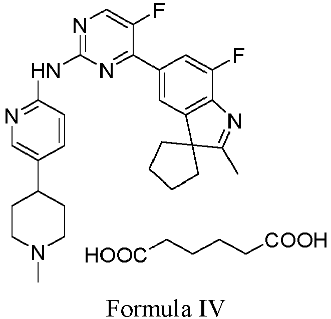

In one embodiment, the adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention comprises one crystal form or multiple crystal forms or can be present in one crystal form or a mixture of multiple crystal forms, and preferably the adipate is present in one crystal form.

In one embodiment, the adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention contains solvent molecules (Preferably with a variable content), preferably the adipate contains water molecules (Preferably with a variable content).

Preferably, the adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine comprises the following crystal form (hereinafter also referred to as adipate crystal form A) or is only present in the following crystal form of the adipate, and the X-ray powder diffraction pattern of the crystal form represented by the diffraction angle of 2θ ± 0.2° at least comprises: characteristic diffraction peaks shown at 4.3°, 8.5°, 15.7°, 21.7°, and 28.2°.

Preferably, the X-ray powder diffraction pattern of the crystal form of the adipate represented by the diffraction angle of 2θ ± 0.2° at least comprises: characteristic diffraction peaks shown at 4.3°, 8.5°, 13.0°, 15.7°, 18.2°, 19.9°, 21.7° and 28.2°.

More preferably, the X-ray powder diffraction pattern of the crystal form of the adipate represented by the diffraction angle of 2θ ± 0.2° at least comprises: characteristic diffraction peaks shown at 4.3°, 8.5°, 13.0°, 15.3°, 15.7°, 18.2°, 19.4°, 19.9°, 21.7° and 28.2°.

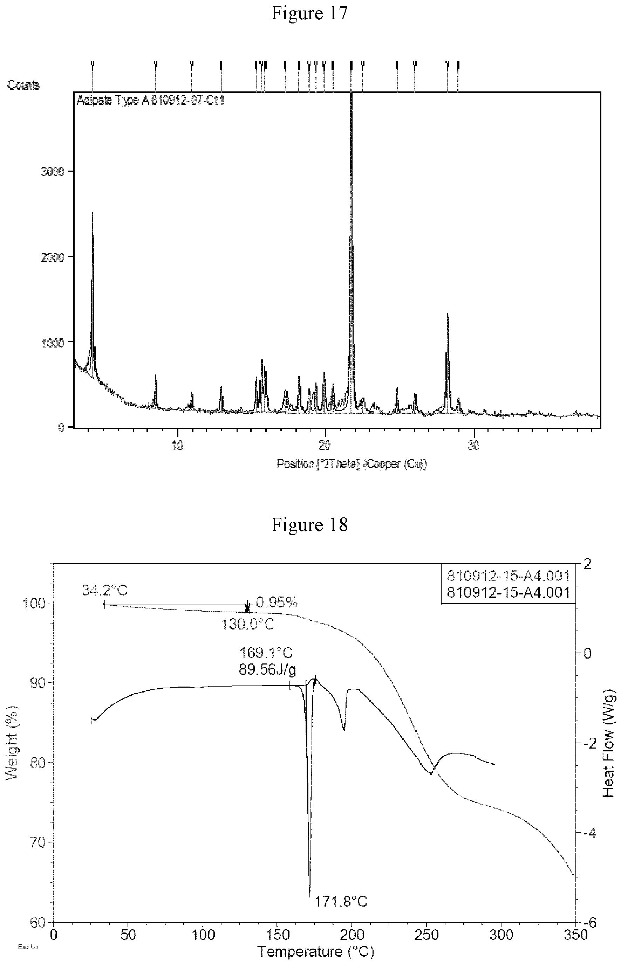

Particularly preferably, the crystal form of the adipate has: XRPD data substantially as shown in Table 6; and/or XRPD pattern substantially as shown in FIG. 17.

The above-mentioned crystal form can be confirmed by X-ray powder diffraction (XRPD), differential scanning calorimetry (DSC), or nuclear magnetic resonance method.

Further, the DSC curve of the above-mentioned adipate crystal form A shows an endothermic peak at 171.8°C.

Further, the above-mentioned adipate crystal form A has a weight loss of 0.95% when heated from room temperature to 130°C.

Preferably, the above-mentioned adipate crystal form A has DSC and TGA curves substantially as shown in FIG. 18.

As the fourth parallel solution of the present application, the present invention provides a succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine.

The succinate has higher solubilities in water and FaSSIF than the free base, and similar solubilities in FaSSIF and FeSSIF, that is, a lower risk of food effects. Moreover, unexpectedly, the succinate has a low hygroscopicity comparable to the free base, and the crystal form is stable without any change after absorption of moisture. At the same time, the succinate has a better physical and chemical stability under high humidity and light illumination conditions.

In one embodiment, the ratio of the succinate ion to the free base in succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine is n:1, wherein n ≥ 0.5, preferably n is 0.5, 1 or 2, more preferably n is 1, for example the mono-succinate represented by the following formula V.

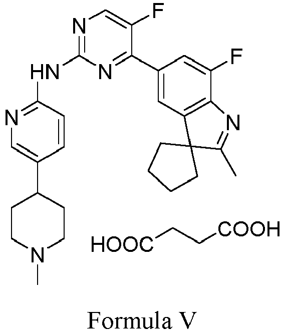

In one embodiment, the succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention comprises one or more crystal forms or can be present in one crystal form or a mixture of multiple crystal forms, and preferably the succinate is present in one crystal form.

In one embodiment, the succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine of the present invention contains solvent molecules(Preferably with a variable content), preferably the succinate contains water molecules (Preferably with a variable content).

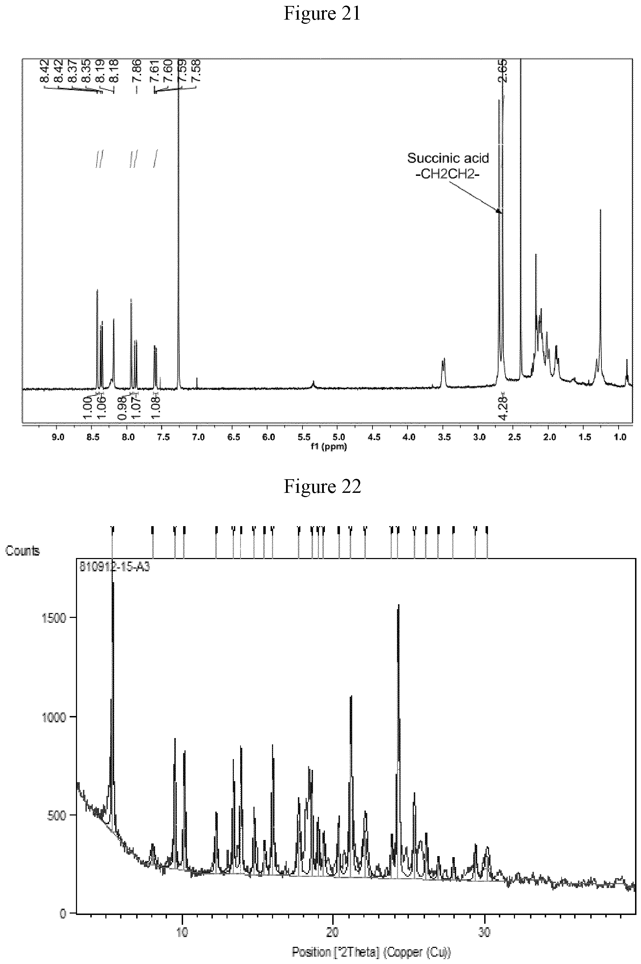

Preferably, the succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine comprises the following crystal form (hereinafter also referred to as succinate crystal form A) or is only present in the following crystal form of the succinate, and the X-ray powder diffraction pattern of the crystal form represented by the diffraction angle of 2θ ± 0.2° at least comprises:
characteristic diffraction peaks shown at 5.4°, 13.9°, 16.0°, 21.1° and 24.3°. Preferably, the X-ray powder diffraction pattern of the crystal form of the succinate represented by the diffraction angle of 2θ ± 0.2° at least comprises:
characteristic diffraction peaks shown at 5.4°, 9.5°, 10.1°, 13.9°, 16.0°, 18.6°, 21.1°, and 24.3°. More preferably, the X-ray powder diffraction pattern of the crystal form of the succinate represented by the diffraction angle of 2θ ± 0.2° at least comprises:
characteristic diffraction peaks shown at 5.4°, 9.5°, 10.1°, 13.4°, 13.9°, 16.0°, 17.7°, 18.6°, 21.1° and 24.3°; or
particularly preferably, the succinate crystal form A has: XRPD data substantially as shown in Table 8; and/or XRPD pattern substantially as shown in FIG. 22.

With regard to the succinate crystal form A:
Further, the DSC curve of the above succinate crystal form A shows endothermic peaks at 192.3°C and 235.9°C; and
Further, the above-mentioned succinate crystal form A has a weight loss of 2.43% when heated from room temperature to 130°C.

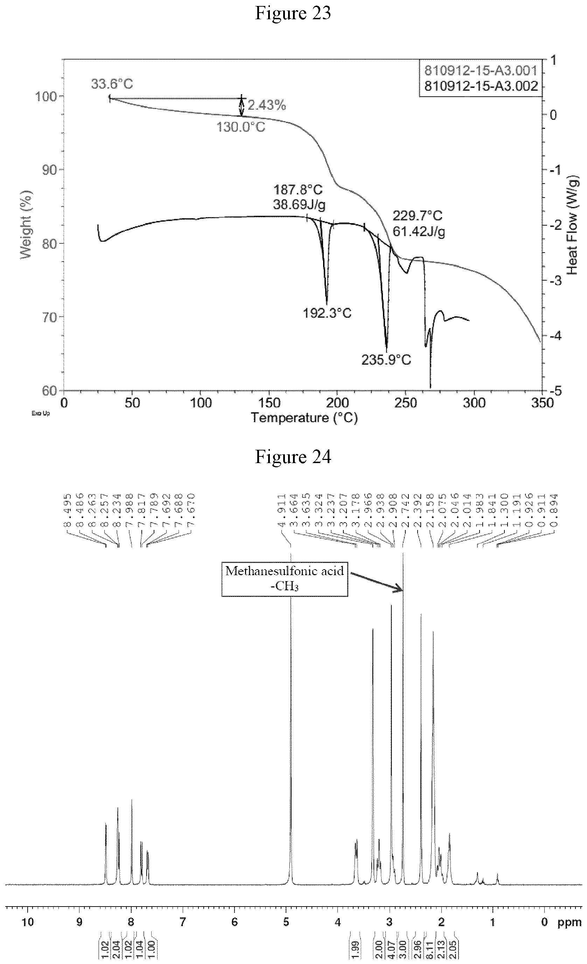

Preferably, the above-mentioned succinate crystal form A has DSC and TGA curves substantially as shown in FIG. 23.

As a particularly preferred technical solution, the present invention also provides the crystal form of the fumarate of the compound represented by formula I:

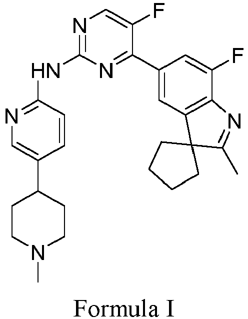

The X-ray powder diffraction pattern of the crystal form of the fumarate represented by the diffraction angle of 2θ ± 0.2° at least comprises:
(1) characteristic diffraction peaks shown at 7.0°, 13.9°, 18.4°, 21.8° and 24.1°; or
(2) characteristic diffraction peaks shown at 4.5°, 10.5°, 18.3°, 20.6° and 23.9°; or
(3) characteristic diffraction peaks shown at 5.9°, 14.4°, 18.6°, 23.6° and 23.9°.

Preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 18.4°, 21.8° and 24.1°; or
(2) characteristic diffraction peaks shown at 4.5°, 8.9°, 10.5°, 17.9°, 18.3°, 20.6°, 22.5°, and 23.9°; or
(3) characteristic diffraction peaks shown at 5.9°, 14.4°, 17.6°, 18.6°, 21.6°, 23.6°, 23.9° and 28.4°.

More preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 15.7 °, 17.2°, 18.4°, 21.8° and 24.1°; or
(2) characteristic diffraction peaks shown at 4.5°, 8.9°, 10.5°, 17.1°, 17.9°, 18.3°, 20.6°, 22.5°, 23.9°, and 27.5°; or
(3) characteristic diffraction peaks shown at 5.9°, 11.7°, 14.4°, 17.6°, 18.6°, 21.6°, 23.6°, 23.9°, 25.0° and 28.4°.

Preferably, the crystal form of the fumarate has XRPD data and/or a XRPD pattern selected from any one of the following (1) to (3):
(1) XRPD data substantially as shown in Table 1; and/or a XRPD pattern substantially as shown in FIG. 2;
(2) XRPD data substantially as shown in Table 2; and/or a XRPD pattern substantially as shown in FIG. 5; and
(3) XRPD data substantially as shown in Table 3; and/or a XRPD pattern substantially as shown in FIG. 8.

Particularly, the X-ray powder diffraction pattern of the fumarate crystal form A represented by the diffraction angle of 2θ ± 0.2° at least comprises:
characteristic diffraction peaks shown at 7.0°, 13.9°, 18.4°, 21.8° and 24.1°; or
characteristic diffraction peaks shown at 5.5°, 7.0°, 9.2°, 10.6°, 13.9°, 18.4°, 21.8° and 24.1°; or
characteristic diffraction peaks shown at 5.5°, 7.0°, 9.2°, 10.6°, 13.9°, 15.7°, 17.2°, 18.4°, 21.8° and 24.1°.

Particularly preferably, the crystal form of the fumarate has:
XRPD data substantially as shown in Table 1; and/or XRPD pattern substantially as shown in FIG. 2.
Further, the DSC curve of the above-mentioned fumarate crystal form A shows an endothermic peak at 254.6°C;
Further, the above-mentioned fumarate crystal form A has a weight loss of 2.50% when heated from room temperature to 180°C;

Preferably, the above-mentioned fumarate crystal form A has DSC and TGA curves substantially as shown in FIG. 3.

The fumarate crystal form A described in the present invention has a relatively high crystallinity and a relatively high melting point, higher solubilities in water and FaSSIF than the free base, and similar solubilities in FaSSIF and FeSSIF, that is, a lower risk of food effects. Moreover, unexpectedly, the fumarate crystal form A has a low hygroscopicity comparable to the free base, and the crystal form is stable without any change after absorption of moisture. At the same time, the fumarate crystal form A has a better physical and chemical stability than the free base under the conditions of high humidity and light illumination.

The present invention also provides a preparation method of the above fumarate crystal form A, comprising allowing 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to react with fumaric acid in a reaction solvent, wherein the reaction solvent is an organic solvent or an aqueous solution of the organic solvent.

According to the preparation method of the present invention, the "5-fluoro-4-(7'-fluoro-2'-methylspiro[cyclopentane-1,3'-indol]-5'-yl)-N-(5-(1-methylpiperid in-4-yl)pyridin-2-yl)pyrimidine-2-amine" as a raw material may be a commercially available crude product, or may also be and particularly preferably the 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine prepared according to the method of Example 17 of CN 106810536. The organic solvent described in the present invention is not particularly limited, as long as the reaction of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine with fumaric acid can be achieved to obtain the fumarate crystal form A. Preferably, the organic solvent is a polar organic solvent. Further preferably, the organic solvent is one or more of alcohol, ketone, halogenated alkane, ether, and ester. More preferably, the organic solvent is one or more of C₁-C₅ linear or branched alkanol, acetone, THF, ethyl acetate, dichloromethane, and chloroform, wherein the C₁-C₅ linear or branched alkanol is preferably methanol, ethanol, propanol or isopropanol. More preferably, the organic solvent is one or more of methanol, ethanol, propanol, isopropanol, acetone, dichloromethane and chloroform.

Preferably, the method further comprises: slowly adding fumaric acid dropwise into 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine for mixing.

Preferably, 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine is allowed to react with fumaric acid at 20°C to a temperature at which the reaction solvent is refluxed.

Preferably, the method further comprises: dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine before reacting with fumaric acid.

Preferably, the solvent used for dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine comprises a first solvent and a second solvent, wherein the first solvent is one or more of ketone, ether, ester and alcohol, preferably one or more alcohols, further preferably the first solvent is one or more of C₁-C₅ linear and branched alkanols, more preferably one or more of methanol, ethanol, propanol and isopropanol; and the second solvent is dichloromethane, chloroform, or a combination of the both.

The mixing volume ratio of the first solvent to the second solvent is not particularly limited, as long as 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine can be dissolved.

Preferably, the mixing volume ratio of the first solvent to the second solvent is 1:(1-10), preferably 1:(1-3), most preferably 1:1, and meanwhile, a particularly ideal combination comprises one or more of methanol, ethanol, propanol and isopropanol used as the first solvent, and dichloromethane, chloroform, or a combination of the both used as the second solvent.

Further preferably, the method further comprises: after dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, subjecting the resulting solution to filtration, so as to remove impurities in 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to ensure the formation of fumarate crystal form A with high quality and high purity.

Further preferably, the method further comprises: evaporating the solvent used for dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine before reacting with fumaric acid.

Further preferably, the method further comprises: cooling the reaction system to a temperature of 10°C to 35°C, preferably cooling to a temperature of 20°C to 30°C after the reaction of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine with fumaric acid is completed, so that the fumarate crystal form A can be fully precipitated.

As a specific preferred mode of the present invention, the method comprises: dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine in a solvent, wherein the solvent is a mixture of the first solvent and the second solvent, wherein the first solvent is methanol, ethanol, propanol or isopropanol; the second solvent is dichloromethane or chloroform, and the volume ratio of the first solvent to the second solvent is 1:(1-3);

The solvent used for dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine is evaporated off to ensure that the fumarate crystal form A can be formed stably.

Fumaric acid is slowly added dropwise into 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine after the above-mentioned solvent is evaporated off, so that the both can react in a solvent, which is methanol, ethanol, propanol or isopropanol, at a reaction temperature of 20°C to a temperature at which the solvent is refluxed.

The reaction system is cooled to 20°C to 30°C after the reaction of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine with fumaric acid is completed, so that the fumarate crystal form A can be fully precipitated.

With the above-mentioned preparation method, the fumarate crystal form A can be repeatedly, stably, and efficiently prepared to ensure crystal quality in the present invention.

Another purpose of the present invention is to provide a pharmaceutical composition, which contains a therapeutically effective amount of at least one of the fumarate, maleate, succinate or adipate of 5-fluoro-4-(7' -fluoro-2'-methylspiro[cyclopentane-1,3'-indol]-5'-yl)-N-(5-(1-methylpiperidi n-4-yl)pyridin-2-yl)pyrimidine-2-amine.

Preferably, the pharmaceutical composition further comprises pharmaceutically acceptable adjuvant, and the pharmaceutically acceptable adjuvant comprises one or more of a carrier, a diluent, and an excipient.

"At least one pharmaceutically acceptable carrier, diluent, or excipient" can be easily selected by a person skilled in the art and determined by the desired mode of administration. Illustrative examples of suitable modes of administration include oral, nasal, parenteral, topical, transdermal, and transrectal modes.

The pharmaceutical composition of the present invention may be in any pharmaceutical form deemed to be suitable by a person skilled in the art. Suitable pharmaceutical forms include solid, semi-solid, liquid, or lyophilized formulations, such as tablets, powders, capsules, suppositories, suspensions, liposomes, and sprays.

Another purpose of the present invention is to provide a kit. The kit comprises at least one of the fumarate, maleate, succinate or adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, and one or more of other biologically active substances. Preferably, the kit further comprises pharmaceutically acceptable adjuvant. More preferably, the pharmaceutically acceptable adjuvant includes at least one of a carrier, a diluent, and an excipient.

Preferably, the other biologically active substances include but are not limited to anticancer agents, immunosuppressive agents, anti-viral agents and the like.

Preferably, the other biologically active substances include an alkylating agent, an antineoplastic antimetabolite drug, a platinum complexing agent, an antibiotic antineoplastic drug, a naturally-derived antineoplastic drug, a hormonal antineoplastic drug, a VEGFR or EGFR inhibitor, an antineoplastic antibody drug, an mTOR inhibitor, a drug for treating brain tumors, and the like.

Preferably, the alkylating agent comprises cyclophosphamide, ifosfamide, thiotepa, semustine, mechlorethamine hydrochloride, busulfan, chlorambucil, melphalan, nitrocaphane, formylmelphalan, carmustine, lomustine, altretamine, dibromomannitol, temozolomide, and the like. The antineoplastic antimetabolite drug comprises cytarabine, fluorouracil, methotrexate, hydroxyurea, tegafur, Meisoindigo, mercaptopurine, and the like. The platinum complexing agent is such as cisplatin, carboplatin, oxaliplatin. The antibiotic antineoplastic drug comprises actinomycin D, mitomycin, doxorubicin, pingyangmycin, epirubicin, pirarubicin, daunorubicin, bleomycin, and the like. The naturally-derived antineoplastic drugs comprises homoharringtonine and its derivatives, vincristine and its derivatives, hydroxycamptothecin and its derivatives, etoposide and its derivatives, vindesine and its derivatives, vinblastine and its derivatives, vinorelbine tartrate, taxol and its derivatives, colchicine and its derivatives, elemene and its derivatives, and the like. The hormonal antineoplastic drug comprises aminoglutethimide, tamoxifen, dexamethasone, dutasteride, flutamide, gonadorelin, leuprolide acetate, letrozole and the like. The VEGFR or EGFR inhibitor comprises sunitinib, sorafenib, imatinib, gefitinib, erlotinib, vandetanib, pazopanib, lapatinib, canertinib, afatinib, mubritinib, dasatinib, neratinib, and the like. The antineoplastic antibody drug comprises trastuzumab, pertuzumab, rituximab, panitumumab, bevacizumab, ipilimumab, ofatumumab, ramucirumab, and the like. The mTOR inhibitor comprises everolimus, sirolimus, zotarolimus, and the like. The drug for treating brain tumors comprises temozolomide and the like.

Further preferably, the salt of the CDK4/6 inhibitor is used in combination with temozolomide to obtain an ideal effect in the treatment of brain tumors.

The present invention also provides the use of the salts of the compound (also called CDK4/6 inhibitor), the pharmaceutical composition or the kit in the preparation of a medicament for the treatment of diseases responsive to the inhibition of a cyclin dependent kinase; and the method for the treatment of diseases responsive to the inhibition of a cyclin dependent kinase using the salt form of the CDK4/6 inhibitor or the pharmaceutical composition.

Preferably, the diseases include but are not limited to a brain tumor, a breast cancer, an urogenital cancer, a lung cancer, a gastrointestinal cancer, an epidermoid cancer, a melanoma, an ovarian cancer, a pancreatic cancer, a neuroblastoma, a head and neck cancer or a bladder cancer, a leukemia, a hyperplasia, a gastric cancer, a colon cancer, a laryngeal cancer, a lymphatic system cancer, a genitourinary tract cancer, a bone cancer, a prostate cancer, small cell lung cancer, a glioma cancer, a colorectal cancer, a kidney cancer, an epithelial cancer, a liver cancer, an esophageal cancer, a hematopoietic system cancer, a lymphoma, a myeloma, a follicular thyroid cancer; a tumor of mesenchymal origin; a tumor of the central or peripheral nervous system; a seminoma; teratocarcinoma; an osteosarcoma; xeroderma pigmentosum; a keratoacanthoma; a follicular thyroid cancer; Kaposi's sarcoma, chronic lymphocytic leukemia, mantle cell lymphoma, and large B-cell lymphoma.

Preferably, the tumor of mesenchymal origin is a fibrosarcoma or a rhabdomyosarcoma. The tumor of the central or peripheral nervous system is an astrocytoma, a neuroblastoma, a glioma, or a schwannomas.

In application, an effective amount of the salt of the above-mentioned CDK4/6 inhibitor is administered to a patient to treat, alleviate or prevent the above-mentioned diseases. Various known methods can be adopted for specific forms, but are not particularly limited thereto in the present invention.

"Treatment" or "alleviation" herein refers to the administration of at least one of the salts of the CDK4/6 inhibitor disclosed herein or other compatible active ingredients to a subject in need thereof, for example, the subject suffering from lung cancer.

"Effective amount" herein refers to such an amount of the salt of the CDK4/6 inhibitor disclosed herein, and this amount can effectively "treat" (as defined above) a disease or dysfunction in a subject.

In the present invention, the "combined product"(or "kit") refers not only to a dosage form containing all components (so-called fixed combined product), and a combined product package containing components separated from one another, but also to separate components applied simultaneously or sequentially, as long as these components are used to prevent and/or treat the same disease.

### Brief Description of the Drawings

Fig. 1 shows the ¹H-NMR spectrum of the fumarate crystal form A in CD₃OD.
Fig. 2 shows the XRPD pattern of the fumarate crystal form A, wherein the "Position [°2theta] (Copper (Cu))" of the abscissa means "Position (2θ) (°) (Copper (Cu))", i.e., the 2θ angle expressed by (°); and the "Counts" refers to the intensity of the diffraction peak (the same below).
Fig. 3 shows the DSC and TGA curves of the fumarate crystal form A, wherein the abscissa represents "Temperature (°C)"; the ordinate on the left represents "Weight (%)", and the ordinate on the right represents "Heat Flow (W/g)" (the same below).
Fig. 4 shows the ¹H-NMR of the fumarate crystal form B in CD₃OD.
Fig. 5 shows the XRPD pattern of the fumarate crystal form B.
Fig. 6 shows the DSC and TGA curves of the fumarate crystal form B.
Fig. 7 shows the ¹H-NMR of the fumarate crystal form C in CD₃OD.
Fig. 8 shows the XRPD pattern of the fumarate crystal form C.
Fig. 9 shows the DSC and TGA curves of the fumarate crystal form C.
Fig. 10 shows the ¹H-NMR of the maleate crystal form A in CD₃Cl.
Fig. 11 shows the XRPD pattern of the maleate crystal form A.
Fig. 12 shows the DSC and TGA curves of the maleate crystal form A.
Fig. 13 shows the ¹H-NMR of the maleate crystal form B in CD₃Cl.
Fig. 14 shows the XRPD pattern of the maleate crystal form B.
Fig. 15 shows the DSC and TGA curves of the maleate crystal form B.
Fig. 16 shows the ¹H-NMR of the adipate crystal form A in CD₃OD.
Fig. 17 shows the XRPD pattern of the adipate crystal form A.
Fig. 18 shows the DSC and TGA curves of the adipate crystal form A.
Fig. 19 shows the XRPD pattern of the free amine crystal form A.
Fig. 20 shows the DSC and TGA curves of the free amine crystal form A.
Fig. 21 shows the ¹H-NMR of the succinate crystal form A in CDCl₃.
Fig. 22 shows the XRPD pattern of the succinate crystal form A.
Fig. 23 shows the DSC and TGA curves of the succinate crystal form A.
Fig. 24 shows the ¹H-NMR of the methanesulfonate crystal form A in CD₃OD.
Fig. 25 shows the XRPD pattern of the methanesulfonate crystal form A.
Fig. 26 shows the DSC and TGA curves of the methanesulfonate crystal form A.
Fig. 27 shows the XRPD pattern of the hydrochloride crystal form B.
Fig. 28 shows the DSC and TGA curves of the hydrochloride crystal form B.
Fig. 29 shows the XRPD comparison patterns of the maleate crystal form B before and after DVS test.
Fig. 30 shows the XRPD comparison patterns of the fumarate crystal form A before and after DVS test.
Fig. 31 shows the XRPD comparison patterns of the succinate crystal form A before and after DVS test.
Fig. 32 shows the XRPD comparison patterns of the adipate crystal form A before and after DVS test.
Fig. 33 shows the XRPD comparison patterns of the hydrochloride crystal form A before and after DVS test.
Fig. 34 shows the XRPD comparison patterns of the methanesulfonate crystal form A before and after DVS test.

### Specific Modes for Carrying Out the Embodiments

The present invention is further described below in conjunction with specific examples. It should be understood that these Examples are only used to illustrate the present invention and not to limit the scope of the present invention. Obviously, the described Examples are only a part of the examples of the present invention, but not all the examples. Based on the Examples of the present invention, all other examples obtained by a person skilled in the art without creative efforts fall within the scope of the present invention.

### Example 1

Preparation of the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

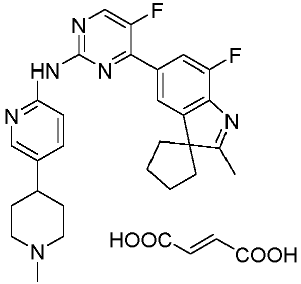

Dichloromethane (22.0 volume) and ethanol (22.0 volume) were added into a reaction kettle under the protection of nitrogen gas at room temperature, 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (2.070 kg) was added while stirring, the temperature was raised to 30°C to 40°C, stirring was performed until 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was completely dissolved, the temperature was cooled to room temperature, and the solution was transferred to a solvent tank for use. Under the protection of nitrogen gas, the above solution was filtered through a microporous filter, transferred to a reaction kettle for stirring, dichloromethane and ethanol were evaporated off at normal pressure, then the temperature of the reaction kettle was maintained at 80 ± 5°C, the ethanol solution (12 volumes) of fumaric acid (1.0 eq) was slowly added dropwise into the reaction kettle through a microporous filter, and the temperature was maintained under stirring overnight. The temperature was lowered to 20°C to 30°C, and the mixture was stirred continuously for at least 1 hour and then centrifuged, and the filter cake was collected. The filter cake was placed in a vacuum oven and dried overnight to obtain 1.605 kg of the fumarate crystal form A of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, with a yield of 63.6%.

¹H-NMR shows that the crystal form is mono-fumarate (Fig. 1). The XRPD pattern is shown in Fig. 2, and the XRPD data is shown in Table 1. DSC data shows that an endothermic peak appears when heated to 254.6°C. TGA data shows that there is a weight loss of 2.50% when heated from room temperature to 180°C (Fig. 3).

**Table 1: X-ray Powder diffraction data of the fumarate crystal form A**

| 2θ (°) | Height [cts] | Rel. Int. [%] |
|---|---|---|
| 5.5 | 507.2 | 20.3 |
| 5.9 | 344.0 | 13.8 |
| 7.0 | 617.6 | 24.8 |
| 9.2 | 593.5 | 23.8 |
| 10.6 | 571.7 | 22.9 |
| 10.9 | 422.6 | 16.9 |
| 13.0 | 241.5 | 9.7 |
| 13.9 | 2056.4 | 82.4 |
| 14.6 | 417.5 | 16.7 |
| 15.7 | 501.5 | 20.1 |
| 16.0 | 321.9 | 12.9 |
| 16.4 | 297.2 | 11.9 |
| 17.2 | 433.7 | 17.4 |
| 17.6 | 319.6 | 12.8 |
| 18.4 | 661.7 | 26.5 |
| 18.6 | 579.3 | 23.2 |
| 19.1 | 379.9 | 15.2 |
| 20.9 | 319.0 | 12.8 |
| 21.8 | 1299.5 | 52.1 |
| 24.1 | 2494.3 | 100.0 |
| 24.9 | 263.3 | 10.6 |
| 25.9 | 342.6 | 13.7 |
| 26.6 | 454.3 | 18.2 |
| 27.0 | 288.5 | 11.6 |
| 30.8 | 236.4 | 9.5 |

### Example 2

Preparation of the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form B) 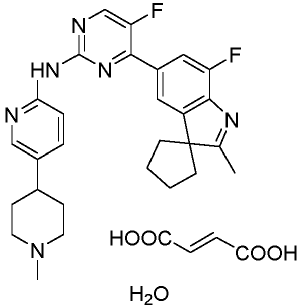 349.7 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was weighed and added into a 20 ml glass flask, 5 ml of THF was added, then a THF(3.75 ml) solution containing 83.4 mg of fumaric acid was added dropwise into the glass flask, the resultant mixture was stirred at room temperature for 4 days, and centrifuged to separate solid out, and the obtained solid was dried at 50°C for 1.5 hours to obtain the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine in crystal form B.

¹H-NMR shows that the crystal form is mono-fumarate (Fig. 4). The XRPD pattern is shown in Fig. 5, and the XRPD data is shown in Table 2. DSC data shows that an endothermic peak appears at 254.4°C. TGA data shows there is a weight loss of 2.71% when heated from room temperature to 130°C (Fig. 6).

**Table 2: X-ray Powder diffraction data of the fumarate crystal form B**

| 2θ (°) | Height [cts] | Rel. Int. [%] |
|---|---|---|
| 4.5 | 885.8 | 100.0 |
| 8.9 | 229.0 | 25.9 |
| 10.5 | 272.8 | 30.8 |
| 14.5 | 181.2 | 20.5 |
| 15.9 | 192.7 | 21.8 |
| 17.1 | 205.0 | 23.1 |
| 17.9 | 213.0 | 24.1 |
| 18.3 | 389.3 | 44.0 |
| 20.6 | 541.1 | 61.1 |
| 21.7 | 106.6 | 12.0 |
| 22.5 | 295.6 | 33.4 |
| 23.9 | 421.0 | 47.5 |
| 24.8 | 159.9 | 18.1 |
| 25.9 | 119.9 | 13.5 |
| 27.5 | 267.5 | 30.2 |
| 31.4 | 118.6 | 13.4 |

### Example 3

Preparation of the fumarate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form C)

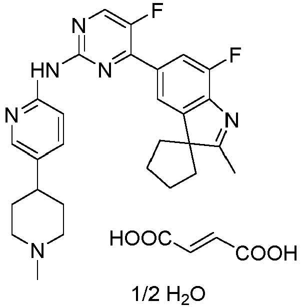

349.7 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was weighed and added into a 20 ml glass flask, 5 ml of isopropanol/water (19:1) was added, then an isopropanol/water (19:1; 3.75 ml) solution containing 83.4 mg of fumaric acid was added dropwise into the glass flask, the resultant mixture was stirred at room temperature for 4 days, and centrifuged to separate solid out, and the obtained solid was dried at 50°C for 1.5 hours to obtain the fumarate crystal form C.

¹H-NMR shows that the crystal form is mono-fumarate (Fig. 7); The XRPD pattern is shown in Fig. 8, and the XRPD data is shown in Table 3. DSC data shows that an endothermic peak begins to appear at 240.2°C. TGA data shows that there is a weight loss of 1.95% when heated from room temperature to 130°C (Fig. 9).

**Table 3: X-ray Powder diffraction data of the fumarate crystal form C**

| 2θ (°) | Height [cts] | Rel. Int. [%] |
|---|---|---|
| 5.9 | 1000.9 | 100.0 |
| 11.7 | 121.2 | 12.1 |
| 14.4 | 286.6 | 28.6 |
| 17.6 | 195.8 | 19.6 |
| 18.6 | 634.9 | 63.4 |
| 21.6 | 152.0 | 15.2 |
| 23.6 | 287.7 | 28.8 |
| 23.9 | 267.4 | 26.7 |
| 25.0 | 147.9 | 14.8 |
| 28.4 | 138.3 | 13.8 |

### Example 4

Preparation of the maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

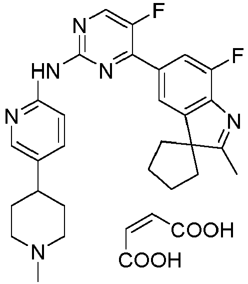

3.5 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine and 8.4 mg of maleic acid were weighed and added into a 5 ml glass flask, 1 ml of THF was added, the resultant mixture was stirred at room temperature for 4 days, and subjected to centrifugal separation , and the solid was dried at 50°C for 5 hours to obtain 4.0 mg of the maleate crystal form A, with a yield of 92.5%.

¹H-NMR shows that the crystal form is mono-maleate (Fig. 10). The XRPD pattern is shown in Fig. 11, and the XRPD data is shown in Table 4. DSC data shows that an endothermic peak appears when heated to 188.3°C. TGA data shows that there is a weight loss of 3.48% when heated from room temperature to 130°C (Fig. 12).

**Table 4: X-ray Powder diffraction data of the maleate crystal form A**

| 2θ (°) | Height [cts] | Rel. Int. [%] |
|---|---|---|
| 7.3 | 187.3 | 38.1 |
| 7.8 | 469.9 | 95.6 |
| 9.0 | 491.6 | 100.0 |
| 13.6 | 101.6 | 20.7 |
| 15.7 | 268.9 | 54.7 |
| 17.9 | 165.1 | 33.6 |
| 18.5 | 181.3 | 36.9 |
| 20.7 | 106.3 | 21.6 |
| 22.7 | 272.7 | 55.5 |
| 24.1 | 111.2 | 22.6 |
| 26.8 | 82.2 | 16.7 |

### Example 5

Preparation of the maleate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form B)

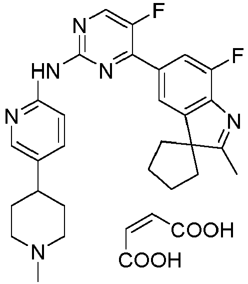

353.5 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was weighed and added into a 20 ml glass flask, 5 ml of IPA/H₂O (19:1, v/v) was added, an IPA/H₂O (19:1, v/v; 3.75 ml) solution containing 83.7 mg of maleic acid was added dropwise into the glass flask, the resultant mixture was stirred at room temperature for 4 days, and subjected to centrifugal separation , and the solid was dried at 50°C for 5 hours to obtain 405.1 mg of maleate crystal form B, with a yield of 92.7%.

¹H-NMR shows that the crystal form is mono-maleate (Fig. 13). The XRPD pattern is shown in Fig. 14, and the XRPD data is shown in Table 5. DSC data shows that an endothermic peak appears when heated to 216.6°C. TGA data shows that there is a weight loss of 3.61% when heated from room temperature to 130°C (Fig. 15).

**Table 5: X-ray Powder diffraction data of the maleate crystal form B**

| 2θ (°) | Height [cts] | Rel. Int. [%] |
|---|---|---|
| 5.7 | 2475.2 | 100.0 |
| 8.5 | 322.4 | 13.0 |
| 11.4 | 253.9 | 10.3 |
| 13.7 | 677.5 | 27.4 |
| 14.1 | 425.5 | 17.2 |
| 17.2 | 465.2 | 18.8 |
| 17.6 | 802.0 | 32.4 |
| 17.9 | 1711.9 | 69.2 |
| 18.9 | 1124.1 | 45.4 |
| 20.5 | 326.0 | 13.2 |
| 21.9 | 465.5 | 18.8 |
| 22.4 | 324.3 | 13.1 |
| 22.9 | 298.8 | 12.1 |
| 23.6 | 1240.8 | 50.1 |
| 23.8 | 797.8 | 32.2 |
| 24.8 | 647.1 | 26.1 |
| 26.1 | 247.5 | 10.0 |
| 26.5 | 387.8 | 15.7 |
| 26.8 | 407.5 | 16.5 |

### Example 6

Preparation of the adipate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

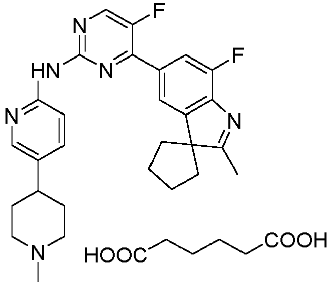

350.6 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was weighed and added into a 20 ml glass flask, 5 ml of THF was added, a tetrahydrofuran solution (3.75 ml) containing 105.4 mg of adipic acid was added dropwise into the glass flask, the resultant mixture was stirred at room temperature for 4 days, and then centrifuged to separate solid out, and the obtained solid was dried at 50°C for 5 hours to obtain 362.4 mg of the adipate crystal form A, with a yield of 79.5%.

¹H-NMR shows that the crystal form is mono-adipate (Fig. 16). The XRPD pattern is shown in Fig. 17, and the XRPD data is shown in Table 6. DSC data shows that an endothermic peak appears when heated to 171.8°C. TGA data shows that there is a weight loss of 0.95% when heated from room temperature to 130°C (Fig. 18).

**Table 6: X-ray Powder diffraction data of the adipate crystal form A**

| 2θ (°) | Height [cts] | Rel. Int. [%] |
|---|---|---|
| 4.3 | 1310.5 | 47.0 |
| 8.5 | 283.3 | 10.2 |
| 13.0 | 226.3 | 8.1 |
| 15.3 | 282.7 | 10.1 |
| 15.7 | 436.1 | 15.6 |
| 15.9 | 391.6 | 14.0 |
| 18.2 | 327.3 | 11.7 |
| 19.4 | 275.9 | 9.9 |
| 19.9 | 357.1 | 12.8 |
| 20.5 | 269.9 | 9.7 |
| 21.7 | 2790.8 | 100.0 |
| 28.2 | 913.2 | 32.7 |

### Comparative Example 1

Preparation of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

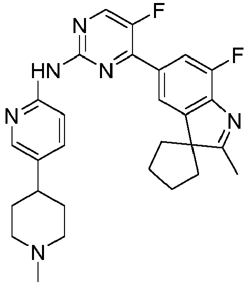

300 g of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine prepared according to the method of Example 17 of CN 106810536 and 3 L of tetrahydrofuran were added into a 10 L reaction kettle, the reaction kettle was heated to 70°C until the solid was completely dissolved, and the reaction was performed for 1 h under refluxing, then cooling was performed slowly to lower the temperature to precipitate solids, the resultant was filtered, and the filter cake was washed with n-hexane, and dried with an infrared lamp to obtain 260 g of a light yellow solid, that is, free amine in crystal form A, with a yield of 86.66%.

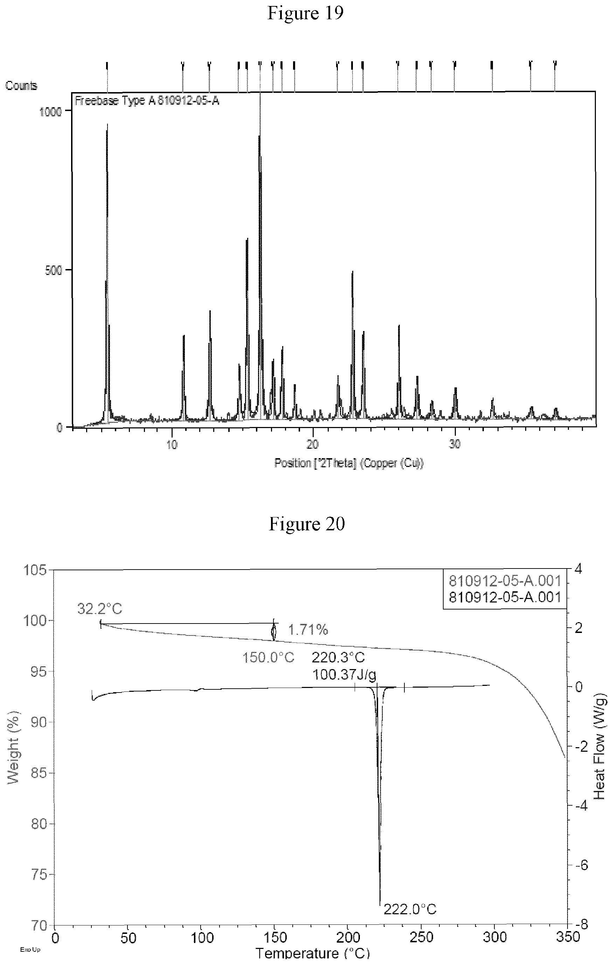

The XRPD pattern is shown in Fig. 19, and the XRPD data is shown in Table 7; DSC data shows that an endothermic peak appears when heated to 222.0°C. TGA data shows that there is a weight loss of 1.71% when heated from room temperature to 150°C (Fig. 20).

**Table 7: X-ray Powder diffraction data of the free amine in crystal form A**

| 2θ (°) | Height [cts] | Rel. Int. [%] |
|---|---|---|
| 5.5 | 649.9 | 88.9 |
| 10.9 | 187.5 | 25.7 |
| 12.7 | 249.1 | 34.1 |
| 14.8 | 125.1 | 17.1 |
| 15.3 | 407.6 | 55.8 |
| 16.3 | 730.9 | 100.0 |
| 17.2 | 138.2 | 18.9 |
| 17.8 | 172.2 | 23.6 |
| 18.7 | 83.9 | 11.5 |
| 21.8 | 103.2 | 14.1 |
| 22.8 | 351.8 | 48.1 |
| 23.5 | 217.0 | 29.7 |
| 26.0 | 229.5 | 31.4 |
| 27.3 | 105.4 | 14.4 |
| 28.4 | 42.9 | 5.9 |
| 30.0 | 77.9 | 10.7 |
| 32.7 | 58.1 | 7.9 |
| 35.4 | 30.1 | 4.1 |
| 37.1 | 27.0 | 3.7 |

### Example 7

Preparation of the succinate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

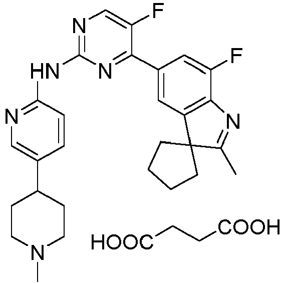

351.6 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine and 5 ml of acetone were added into a reaction flask, magnetically stirred, then added with 3.75 ml of an acetone solution containing 85.1 mg of succinic acid dropwise, and stirred at room temperature for four days. The reaction mixture was centrifuged to separate solid out, and the solid was dried at 50°C for 5 hours to obtain 377.7 mg of solid, i.e., succinate crystal form A, with a yield of 86.5%.

¹H-NMR shows that the crystal form is mono-succinate (Fig. 21). The XRPD pattern is shown in Fig. 22, and the XRPD data is shown in Table 8. DSC data shows that endothermic peaks appear at 192.3°C and 235.9°C. TGA data shows that there is a weight loss of 2.4% when heated from room temperature to 130°C (Fig. 23).

**Table 8: X-ray Powder diffraction data of the succinate crystal form A**

| 2θ (°) | Height [cts] | Rel. Int. [%] |
|---|---|---|
| 5.4 | 964.3 | 87.7 |
| 8.0 | 72.4 | 6.6 |
| 9.5 | 451.7 | 41.1 |
| 10.1 | 432.1 | 39.3 |
| 12.3 | 219.2 | 19.9 |
| 13.4 | 417.6 | 38.0 |
| 13.9 | 460.9 | 41.9 |
| 14.8 | 258.0 | 23.5 |
| 15.5 | 120.9 | 11.0 |
| 16.0 | 487.2 | 44.3 |
| 17.7 | 281.0 | 25.6 |
| 18.6 | 420.7 | 38.2 |
| 19.0 | 231.8 | 21.1 |
| 19.4 | 167.6 | 15.2 |
| 20.3 | 249.4 | 22.7 |
| 21.1 | 676.5 | 61.5 |
| 22.1 | 229.1 | 20.8 |
| 23.8 | 155.3 | 14.1 |
| 24.3 | 1100.1 | 100.0 |
| 25.3 | 328.3 | 29.9 |
| 26.1 | 178.0 | 16.2 |
| 27.0 | 90.7 | 8.2 |
| 27.9 | 90.4 | 8.2 |
| 29.4 | 142.3 | 12.9 |
| 30.2 | 122.4 | 11.1 |

### Comparative Example 2

Preparation of the methanesulfonate of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form A)

61.1 mg of methanesulfonic acid and 305.3 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine were added into a reaction flask, 10 ml of ethyl acetate was added, the resultant mixture was stirred at room temperature for 3 days, and centrifuged to separate solid out, and the solid was dried at 50°C for 3 hours to obtain 370.2 mg of solid, i.e., the methanesulfonate crystal form A, with a yield of 101.0%.

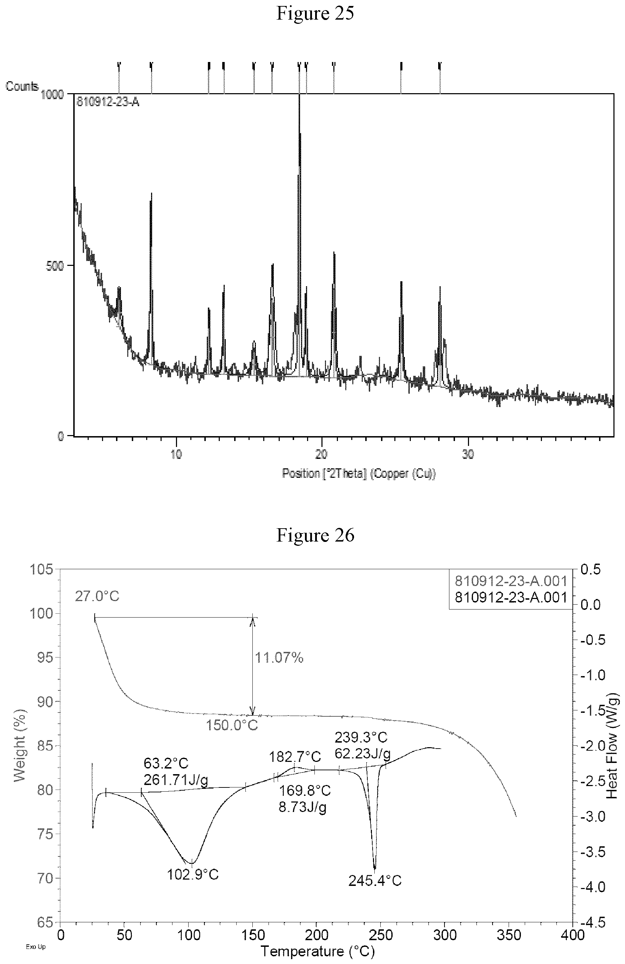

¹H-NMR shows that the crystal form is mono-methanesulfonate (Fig. 24). The XRPD pattern is shown in Fig. 25, and the XRPD data is shown in Table 9. DSC data shows that a wide endothermic peak appears when heated to 102.9°C. TGA data shows that there is a weight loss of 11.07% when heated from room temperature to 150°C (Fig. 26).

**Table 9: X-ray Powder diffraction data of the methanesulfonate crystal form A**

| 2θ (°) | Height [cts] | Rel. Int. [%] |
|---|---|---|
| 6.1 | 79.1 | 12.8 |
| 8.3 | 342.7 | 55.5 |
| 12.3 | 133.6 | 21.6 |
| 13.3 | 193.9 | 31.4 |
| 15.3 | 67.6 | 11.0 |
| 16.6 | 222.3 | 36.0 |
| 18.4 | 617.1 | 100.0 |
| 18.9 | 201.9 | 32.7 |
| 20.8 | 259.9 | 42.1 |
| 25.4 | 212.8 | 34.5 |
| 28.0 | 240.3 | 38.9 |

### Comparative Example 3

Preparation of the hydrochloride of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine (crystal form B)

352.2 mg of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine was weighed and added into a 20 ml glass flask, and 5 ml of THF was added. 60 µl of hydrochloric acid was weighed and diluted with 3.75 ml of THF, then the obtained acid solution was gradually added into a free base suspension, and the resultant mixture was stirred at room temperature for 4 days, and centrifuged to separate solid out, and the solid was dried at 50°C for 5 hours. 361.4 mg of solid was collected to obtain the hydrochloride crystal form B (yield: 83.3%).

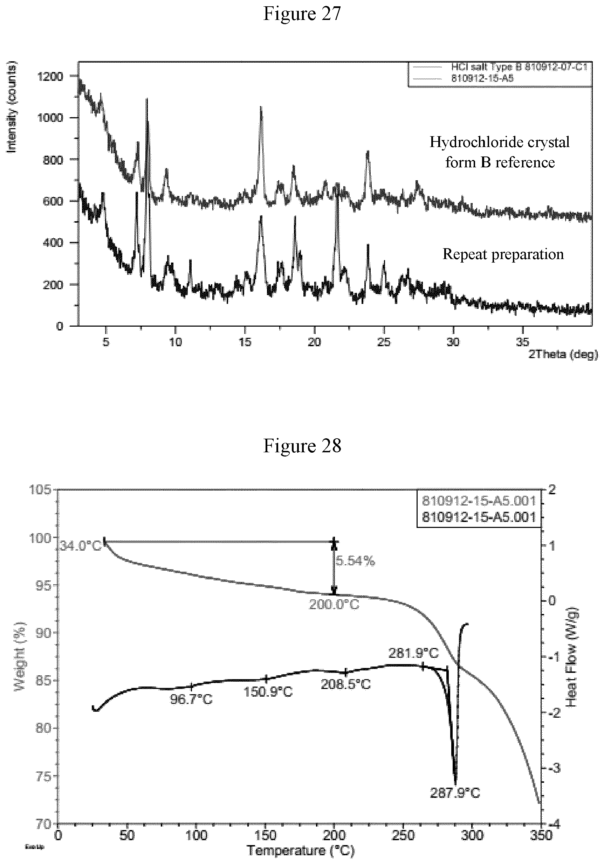

The XRPD pattern is shown in Fig. 27. DSC data shows that there are three endothermic peaks before melting at 281.9°C (starting temperature). TGA data shows that there is a weight loss of 5.5% before 200°C (Fig. 28).

### Experimental Example 1

The purpose of the present Experimental Example is to compare the solubilities of various types of salts (also referred to as salt forms) obtained in the present invention with those of the crystal forms/salt forms prepared in the Comparative Examples.

Experimental method: the solubility test was carried out in three solvents including water, fasted state simulated intestinal fluid (FaSSIF) and fed state simulated intestinal fluid (FeSSIF), to evaluate the solubilities of the salts of the present invention.

In the experiment, the sample and the solvent were mixed in a centrifuge tube (the initial feeding amount was 10 mg/ml), then the centrifuge tube was sealed and fixed on a rotating disk with a rotation speed of 25 rpm, and sampling was performed after rotating and mixing for 24 h at 37°C. The turbid sample was subjected to centrifugal separation, and the filtered supernatant was measured for HPLC concentration. If the sample solution was clear, the concentration of the obtained solution was measured.

Experimental objects: free base crystal form A, maleate crystal form B, fumarate crystal form A, fumarate crystal form C, adipate crystal form A, and succinate crystal form A.

Experimental results: see Tables 10 to 12, the solubilities of different salt forms and crystal forms in water, FaSSIF and FeSSIF.

**Table 10: Solubilities of different salt forms and crystal forms in water**

| Sample name | Solubility (mg/ml) |
|---|---|
| Free base crystal form A | 0.075 |
| Maleate crystal form B | 12.9 |
| Fumarate crystal form A | 6.3 |
| Fumarate crystal form C | 0.48 |
| Adipate crystal form A | 6.5 |
| Succinate crystal form A | 7.5 |

As shown in Table 10, the solubilities of the maleate crystal form B, the fumarate crystal form A, the fumarate crystal form C, the adipate crystal form A, and the succinate crystal form A in water are 12.9 mg/ml, 6.3 mg/ml, 0.48 mg/ml, 6.5 mg/ml, and 7.5 mg/ml, respectively, which are significantly higher than the solubility (0.075 mg/ml) of the free base crystal form A in water.

**Table 11: Solubilities of different salt forms and crystal forms in FaSSIF**

| Sample name | Solubility (mg/ml) |
|---|---|
| Free base crystal form A | 0.19 |
| Maleate crystal form B | 7.1 |
| Fumarate crystal form A | 7.6 |
| Fumarate crystal form C | 5.9 |
| Adipate crystal form A | 6.8 |
| Succinate crystal form A | 6.4 |

**Table 12: Solubilities of different salt forms and crystal forms in FeSSIF**

| Sample name | Solubility (mg/ml) |
|---|---|
| Free base crystal form A | 10.2 |
| Maleate crystal form B | 7.8 |
| Fumarate crystal form A | 8.0 |
| Fumarate crystal form C | 13.6 |
| Adipate crystal form A | 11.6 |
| Succinate crystal form A | 10.7 |

As shown in Table 11, the solubilities of the maleate crystal form B, the fumarate crystal form A, the fumarate crystal form C, the adipate crystal form A, and the succinate crystal form A in FaSSIF are significantly higher than the solubility (0.19 mg/ml) of the free base crystal form A in FaSSIF, respectively.

As shown in Table 12, the solubilities of the maleate crystal form B, the fumarate crystal form A, the fumarate crystal form C, the adipate crystal form A, the succinate crystal form A in FeSSIF are comparable to the solubility (10.2 mg/ml) of the free base crystal form A in FeSSIF, and the solubilities of the above-mentioned salts in the fasted state simulated intestinal fluid (FaSSIF) are approximate to those in the fed state simulated intestinal fluid (FeSSIF), suggesting a lower risk of food effects in comparison with the free base crystal form A.

### Experimental Example 2

In the present Experimental Example, the hygroscopicities of the various salt forms provided by the present invention were compared with those of the crystal forms/salt forms prepared in the Comparative Examples.

The moisture adsorption capacities of the representative salts and the free base of the above Examples and Comparative Examples were tested when the humidity reached 80% RH through a dynamic moisture adsorption (DVS) test at 25°C, so as to compare the hygroscopicities.

Experimental objects: free base crystal form A, maleate crystal form B, fumarate crystal form A, adipate crystal form A, succinate crystal form A, methanesulfonate crystal form A, and hydrochloride crystal form B.

Experimental results: the test results are shown in Table 13, wherein, > 15% means very hygroscopic, 2% to 15% means hygroscopic, 0.2% to 2% means slightly hygroscopic, and <0.2% means almost non-hygroscopic.

**Table 13: Hygroscopicities of different salt forms and crystal forms**

| Sample name | Hygroscopicity (%) | Whether the crystal form has a change |
|---|---|---|
| Free base crystal form A | 0.10 | No change |
| Maleate crystal form B | 2.00 | No change |
| Fumarate crystal form A | 0.35 | No change |
| Adipate crystal form A | 0.22 | No change |
| Succinate crystal form A | 0.33 | No change |
| Methanesulfonate crystal form A | 12.02 | Change |
| Hydrochloride crystal form B | 9.12 | Change |

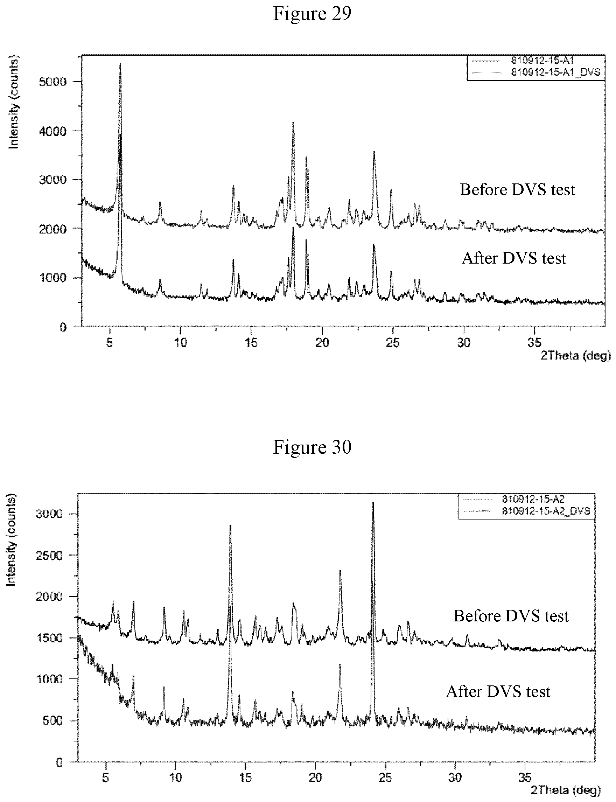

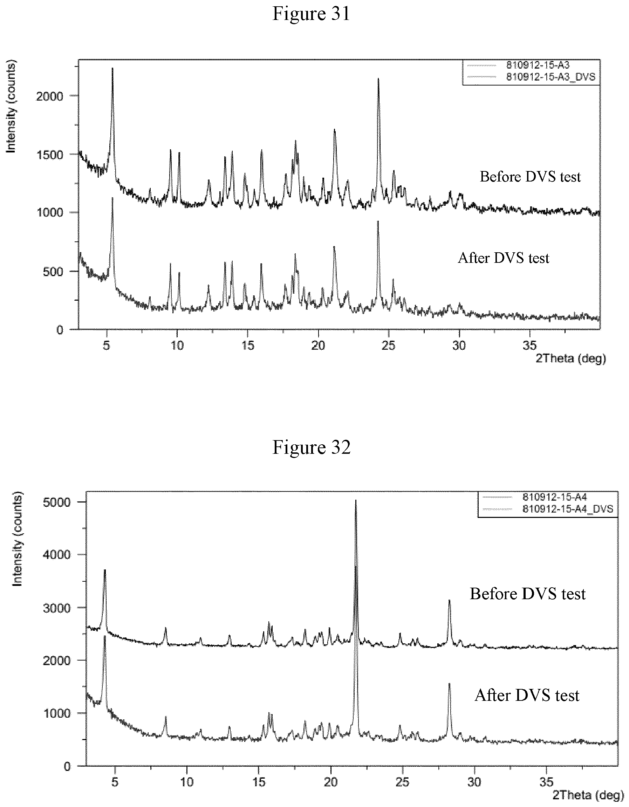

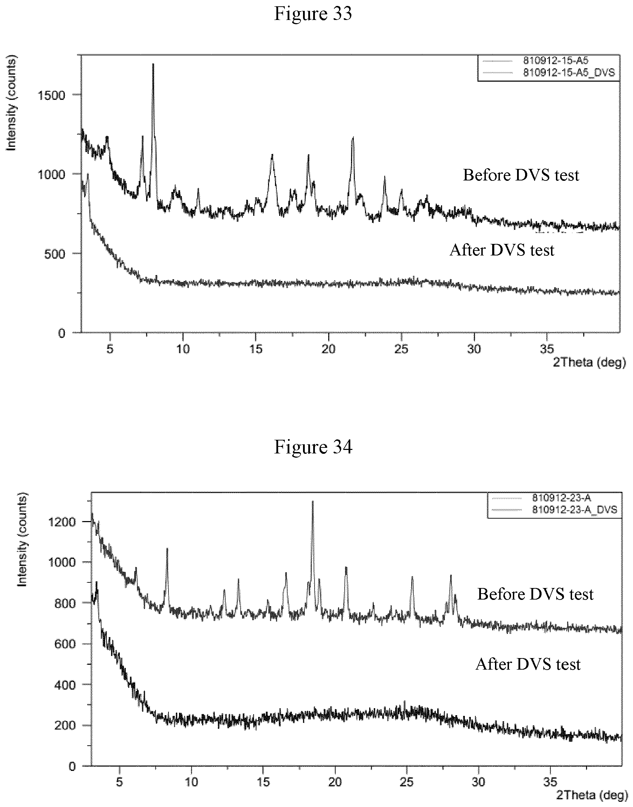

The results show (see Figures 29 to 34): under the conditions of 25°C/80% RH, the moisture adsorption capacities of the maleate crystal form B, the fumarate crystal form A, the succinate crystal form A and the adipate crystal form A are 0.10% to 2.00%, which means a slight hygroscopicity and almost equivalents to the that of the free base, and the crystal forms of these samples had no change before and after the DVS test, showing a good physical stability under the corresponding test conditions; however, under the conditions of 25°C/80% RH, the moisture adsorption capacities of the hydrochloride crystal form B and the methanesulfonate crystal form A are 9.12% and 12.02%, respectively, which means the hygroscopicities are relatively high, and these samples changed to amorphous after the DVS test.

### Experimental Example 3

In the present Experimental Example, the stabilities of the various salt forms provided by the present invention were compared with those of the crystal forms/salt forms prepared in the Comparative Examples.

Experimental method: appropriate amount of samples were respectively weighed and placed in open containers under the conditions of light illumination of 4500 lux, 25°C/60% RH and 40°C/75% RH to perform solid-state stability test evaluation for one week. One week later, the purities of the test samples were tested by HPLC to detect the change of purity. Three groups of parallel tests were set for each test condition.

Experimental objects: free base crystal form A, maleate crystal form B, fumarate crystal form A, fumarate crystal form C, adipate crystal form A, succinate crystal form A, and methanesulfonate crystal form A.

Experimental results: the test results are shown in Table 14.

**Table 14: Stabilities of different salt forms and crystal forms**

| **Crystal form** | **Initial purity (Area%)** | **Test conditions** | **HPLC purity 1 week** | |
|---|---|---|---|---|
| | | | **Area%** | **Relative to the initial purity%** |
| Free base crystal form A | 100.00 | Light illumination 4500 lux | 99.28 | 99.3 |
| | | 25°C/60%RH | 100.0 | 100.0 |
| | | 40°C/75%RH | 99.92 | 99.9 |
| Maleate crystal form B | 99.91 | Light illumination 4500 lux | 99.83 | 99.9 |
| | | 25°C/60%RH | 99.88 | 100.0 |
| | | 40°C/75%RH | 99.89 | 100.0 |
| Fumarate crystal form A | 99.92 | Light illumination 4500 lux | 99.89 | 100.0 |
| | | 25°C/60%RH | 99.89 | 100.0 |
| | | 40°C/75%RH | 99.88 | 100.0 |
| Fumarate crystal form C | 99.48 | Light illumination 4500 lux | 99.46 | 100.0 |
| | | 25°C/60%RH | 99.48 | 100.0 |
| | | 40°C/75%RH | 99.48 | 100.0 |
| Adipate crystal form A | 99.90 | Light illumination 4500 lux | 99.88 | 100.0 |
| | | 25°C/60%RH | 99.88 | 100.0 |
| | | 40°C/75%RH | 99.89 | 100.0 |
| Succinate crystal form A | 99.94 | Light illumination 4500 lux | 99.90 | 100.0 |
| | | 25°C/60%RH | 99.90 | 100.0 |
| | | 40°C/75%RH | 99.90 | 100.00 |
| Methanesulfonate crystal form A | 99.81 | Light illumination 4500 lux | 98.28 | 98.5 |
| | | 25°C/60%RH | 99.73 | 99.9 |
| | | 40°C/75%RH | 99.16 | 99.4 |

As shown in Table 14, after the free base crystal form A, the maleate crystal form B, the fumarate crystal form A, the fumarate crystal form C, the adipate crystal form A, the succinate crystal form A, and the methanesulfonate crystal form A were placed under light illumination of 4500 lux for one week, the HPLC purities relative to the initial purity are 99.3%, 99.9%, 100%, 100%, 100%, 100%, and 98.5%, respectively, therefore, the maleate crystal form B, the fumarate crystal form A, the adipate crystal form A, the succinate crystal form A are more stable than the free amine crystal form A, but the methanesulfonate crystal form A is more unstable than the free amine crystal form A. Meanwhile, the maleate crystal form B, the fumarate crystal form A, the fumarate crystal form C, the adipate crystal form A and the succinate crystal form A also have a high stability at 40°C/75% RH, and all show a HPLC purity of 100% relative to the initial purity after placed for one week, while the HPLC purity relative to the initial purity of the methanesulfonate crystal form A is 99.4%, therefore, under the conditions of 40°C/75% RH, the stabilities of the maleate crystal form B, the fumarate crystal form A, the fumarate crystal form C, the adipate crystal form A and the succinate crystal form A are better than or comparable to the stability of the free base, but the methanesulfonate crystal form A is more unstable than the free base crystal form A.

### Instrument information and method involved in the present invention

### (1) X-ray powder diffraction (XRPD)

The XRPD patterns were collected through reflection mode on a PANalytacal Empyrean and Bruker X-ray powder diffraction analyzer. The XRPD test parameters were shown in Table 15.

**Table 15: XRPD test parameters**

| **Parameters** | **PANalytical** | **PANalytical** | **Bruker** |
|---|---|---|---|
| Mode | Empyrean | X' Pert³ | D2 PHASER |
| X-ray | Cu, kα, Kα1 (Å) : 1.540598, Kα2 (Å) : 1.544426 Kα2/Kα1 intensity ratio : 0.50 | Cu, kα, Kα1 (Å) : 1.540598, Kα2(Å) : 1.544426 Kα2/Kα1 intensity ratio : 0.50 | Cu, kα, Kα1 (Å) : 1.5406, Kα2(Å) : 1.54439 Kα2/Kα1 intensity ratio : 0.50 |
| X-ray tube setting | 45 kV, 40 mA | 45 kV, 40 mA | 30 kV, 10 mA |
| Divergence slit | Automatic | 1/8° | 0.6 mm |
| Scanning mode | Continuous | Continuous | Continuous |
| Scanning range (°2Theta) | 3° to 40° | 3° to 40° | 3° to 40° |
| Step length time of scanning (s) | 17.8 | 46.7 | 0.1 |
| Step length of scanning (°2Theta) | 0.0167 | 0.0263 | 0.0201 |
| Scanning time | 5 minutes and 30 seconds | 5 minutes and 4 seconds | 3 minutes and 27 seconds |

### (2) Thermogravimetric analysis (TGA) and differential scanning calorimetry (DSC)

TGA and DSC curves were collected on a TA Q500/5000 thermogravimetric analyzer and a TA Q200/2000 differential scanning calorimeter, respectively. The test parameters were listed in Table 16.

**Table 16: DSC and TGA test parameters**

| **Parameters** | **TGA** | **DSC** |
|---|---|---|
| Method | Linear heating | Linear heating |
| Sample tray | Platinum tray, open | Aluminum tray, covered |
| Temperature range | Room temperature to target temperature | 25°C to target temperature |
| Scanning rate (°C/min) | 10 | |
| Protecting gas | Nitrogen gas | |

### (3) High performance liquid chromatography (HPLC)

High performance liquid chromatograms were collected on an Agilent 1100 HPLC. The specific instrument parameters and test parameters were shown in Table 17.

**Table 17: HPLC purity test parameters**

| **Parameters** | **Set value** | |
|---|---|---|
| Chromatographic column | Phenomenex Gemini C18 110A (4.6 × 150 mm, 3 µm) | |
| Mobile phase | A: aqueous solution of 20 mmol NH₄Ac (pH 9.0) | |
| | B: Acetonitrile | |
| Gradient | Time (min) | % B |
| | 0.0 | 35 |
| | 15.0 | 40 |
| | 25.0 | 100 |
| | 28.0 | 100 |
| | 28.1 | 35 |
| | 30.0 | 35 |
| Time | 30.0 min | |
| Post running time | 3.0 min | |
| Flow rate | 1.0 ml/min | |
| Injection volume | 5 µl | |
| Detection wavelength | 272 nm | |
| Column temperature | 40°C | |
| Sample chamber temperature | Room temperature | |
| Diluting solution | Acetonitrile: Water = 1:1 | |

### (4) Solution NMR

The solution nuclear magnetic spectrums were collected on a Bruker 400M nuclear magnetic resonance instrument.

Although the present invention has been described in detail with general description and specific embodiments above, it is obvious to a person skilled in the art that some modifications or improvements can be made on the basis of the present invention. Therefore, these modifications or improvements made on the basis of not deviating from the spirit of the present invention belong to the scope claimed by the present invention.

## Claims

1. A salt of the compound represented by Formula I: 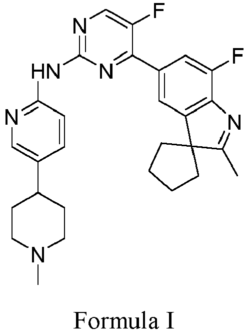 the salt is selected from fumarate, maleate, adipate or succinate.

2. The salt according to claim 1, wherein the molar ratio of fumarate ion, maleate ion, adipate ion or succinate ion to free base in the salt is each independently n:1, wherein n ≥ 0.5, preferably n is 0.5, 1 or 2, more preferably n is 1;
and/or, the salt comprises one or more crystal forms, preferably the salt is present in one crystal form or a mixture of multiple crystal forms, particularly preferably the salt is present in one crystal form; and/or, the salt contains solvent molecules , preferably the salt contains solvent molecules with a variable content, further preferably the salt contains water molecules, preferably water molecules with a variable content.

3. The salt according to claim 1 or 2, wherein the fumarate comprises one or more of the following crystal forms of fumarate, preferably the fumarate is present in any one of the following crystal forms of fumarate or a mixture of multiple crystal forms of the fumarate, and particularly preferably the fumarate is any one of the following crystal forms of fumarate:
the X-ray powder diffraction pattern of the crystal form of the fumarate represented by the diffraction angle of 2θ ± 0.2° at least comprises:
(1) characteristic diffraction peaks shown at 7.0°, 13.9°, 18.4°, 21.8° and 24.1°; or
(2) characteristic diffraction peaks shown at 4.5°, 10.5°, 18.3°, 20.6° and 23.9°; or
(3) characteristic diffraction peaks shown at 5.9°, 14.4°, 18.6°, 23.6° and 23.9°;
preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 18.4°, 21.8° and 24.1°; or
(2) characteristic diffraction peaks shown at 4.5°, 8.9°, 10.5°, 17.9°, 18.3°, 20.6°, 22.5°, and 23.9°; or
(3) characteristic diffraction peaks shown at 5.9°, 14.4°, 17.6°, 18.6°, 21.6°, 23.6°, 23.9° and 28.4°;
more preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 15.7°, 17.2°, 18.4°, 21.8° and 24.1°; or
(2) characteristic diffraction peaks shown at 4.5°, 8.9°, 10.5°, 17.1°, 17.9°, 18.3°, 20.6°, 22.5°, 23.9°, and 27.5°; or
(3) characteristic diffraction peaks shown at 5.9°, 11.7°, 14.4°, 17.6°, 18.6°, 21.6°, 23.6°, 23.9°, 25.0° and 28.4°;
and particularly preferably, the crystal form of the fumarate has XRPD data and/or a XRPD pattern selected from any one of the following (1) to (3):
(1) XRPD data substantially as shown in Table 1; and/or a XRPD pattern substantially as shown in FIG. 2;
(2) XRPD data substantially as shown in Table 2; and/or a XRPD pattern substantially as shown in FIG. 5; and
(3) XRPD data substantially as shown in Table 3; and/or a XRPD pattern substantially as shown in FIG. 8.

4. The salt according to claim 1 or 2, wherein the maleate comprises one or more of the following crystal forms of maleate, preferably the maleate is present in any one of the following crystal forms of maleate or a mixture of multiple crystal forms of the maleate, particularly preferably the maleate is any one of the following crystal forms of maleate:
the X-ray powder diffraction pattern of the crystal form of the maleate represented by the diffraction angle of 2θ ± 0.2° at least comprises:
(1) characteristic diffraction peaks shown at 7.8°, 9.0°, 15.7°, 18.4° and 22.7°; or
(2) characteristic diffraction peaks shown at 5.7°, 13.7°, 17.9°, 18.9° and 23.6°;
preferably, the X-ray powder diffraction pattern of the crystal form of the maleate represented by the diffraction angle of 2θ ± 0.2° at least comprises:
(1) characteristic diffraction peaks shown at 7.3°, 7.8°, 9.0°, 15.7°, 17.9°, 18.4°, 20.7° and 22.7°; or
(2) characteristic diffraction peaks shown at 5.7°, 13.7°, 17.6°, 17.9°, 18.9°, 21.9°, 23.6° and 24.8°;
more preferably, the X-ray powder diffraction pattern of the crystal form of the maleate represented by the diffraction angle of 2θ ± 0.2° at least comprises:
(1) characteristic diffraction peaks shown at 7.3°, 7.8°, 9.0°, 13.6°, 15.7°, 17.9°, 18.4°, 20.7°, 22.7°, and 24.1°; or
(2) characteristic diffraction peaks shown at 5.7°, 13.7°, 14.1°, 17.2°, 17.6°, 17.9°, 18.9°, 21.9°, 23.6° and 24.8°;
particularly preferably, the crystal form of the maleate has XRPD data and/or a XRPD pattern selected from any one of the following (1) to (2):
(1) XRPD data substantially as shown in Table 4; and/or a XRPD pattern substantially as shown in FIG. 11; and
(2) XRPD data substantially as shown in Table 5; and/or a XRPD pattern substantially as shown in FIG. 14.

5. The salt according to claim 1 or 2, wherein the adipate comprises the following crystal forms of adipate or is only present in the following crystal forms of adipate, the X-ray powder diffraction pattern of the crystal form of the adipate represented by the diffraction angle of 2θ ± 0.2° at least comprises: characteristic diffraction peaks shown at 4.3°, 8.5°, 15.7°, 21.7° and 28.2°;
preferably, the X-ray powder diffraction pattern of the crystal form of the adipate represented by the diffraction angle of 2θ ± 0.2° at least comprises: characteristic diffraction peaks shown at 4.3°, 8.5°, 13.0°, 15.7°, 18.2°, 19.9°, 21.7° and 28.2°;
more preferably, the X-ray powder diffraction pattern of the crystal form of the adipate represented by the diffraction angle of 2θ ± 0.2° at least comprises: characteristic diffraction peaks shown at 4.3°, 8.5°, 13.0°, 15.3°, 15.7°, 18.2°, 19.4°, 19.9°, 21.7° and 28.2°;
particularly preferably, the crystal form of the adipate has XRPD data substantially as shown in Table 6, and/or, a XRPD pattern substantially as shown in FIG. 17.

6. The salt according to claim 1 or 2, wherein the succinate comprises the following crystal forms of succinate or is only present in the following crystal forms of succinate, the X-ray powder diffraction pattern of the crystal form of the succinate represented by the diffraction angle of 2θ ± 0.2° at least comprises:
characteristic diffraction peaks shown at 5.4°, 13.9°, 16.0°, 21.1° and 24.3°;
preferably, the X-ray powder diffraction pattern of the crystal form of the succinate represented by the diffraction angle of 2θ ± 0.2° at least comprises:
characteristic diffraction peaks shown at 5.4°, 9.5°, 10.1°, 13.9°, 16.0°, 18.6°, 21.1° and 24.3°;
more preferably, the X-ray powder diffraction pattern of the crystal form of the succinate represented by the diffraction angle of 2θ ± 0.2° at least comprises:
characteristic diffraction peaks shown at 5.4°, 9.5°, 10.1°, 13.4°, 13.9°, 16.0°, 17.7°, 18.6°, 21.1° and 24.3°;
particularly preferably, the crystal form of the succinate has XRPD data substantially as shown in Table 8, and/or a XRPD pattern substantially as shown in FIG. 22.

7. A crystal form of the fumarate of the compound represented by formula I: 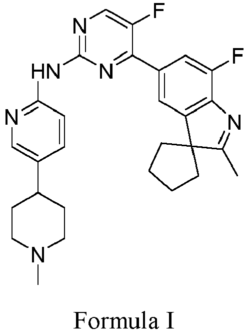
wherein, the X-ray powder diffraction pattern of the crystal form of the fumarate represented by the diffraction angle of 2θ ± 0.2° at least comprises:
(1) characteristic diffraction peaks shown at 7.0°, 13.9°, 18.4°, 21.8° and 24.1°; or
(2) characteristic diffraction peaks shown at 4.5°, 10.5°, 18.3°, 20.6° and 23.9°; or
(3) characteristic diffraction peaks shown at 5.9°, 14.4°, 18.6°, 23.6° and 28.4°;
preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 18.4°, 21.8° and 24.1°;
(2) characteristic diffraction peaks shown at 4.5°, 8.9°, 10.5°, 17.9°, 18.3°, 20.6°, 22.5°, and 23.9°; or
(3) characteristic diffraction peaks shown at 5.9°, 14.4°, 17.6°, 18.6°, 21.6°, 23.6°, 23.9° and 28.4°;
more preferably, the X-ray powder diffraction pattern of the crystal form of the fumarate represented by the diffraction angle of 2θ ± 0.2° at least comprises:
(1) characteristic diffraction peaks shown at 5.5°, 7.0°, 9.2°, 10.6°, 13.9°, 15.7°, 17.2°, 18.4°, 21.8° and 24.1°; or
(2) characteristic diffraction peaks shown at 4.5°, 8.9°, 10.5°, 17.1°, 17.9°, 18.3°, 20.6°, 22.5°, 23.9°, and 27.5°; or
(3) characteristic diffraction peaks shown at 5.9°, 11.7°, 14.4°, 17.6°, 18.6°, 21.6°, 23.6°, 23.9°, 25.0° and 28.4°;
particularly preferably, the crystal form of the fumarate has XRPD data and/or a XRPD pattern selected from any one of the following (1) to (3):
(1) XRPD data substantially as shown in Table 1; and/or a XRPD pattern substantially as shown in FIG. 2;
(2) XRPD data substantially as shown in Table 2; and/or a XRPD pattern substantially as shown in FIG. 5; and
(3) XRPD data substantially as shown in Table 3; and/or a XRPD pattern substantially as shown in FIG. 8.

8. The crystal form of the fumarate according to claim 7, wherein the X-ray powder diffraction pattern of the crystal form of the fumarate represented by the diffraction angle of 2θ ± 0.2° at least comprises:
characteristic diffraction peaks shown at 7.0°, 13.9°, 18.4°, 21.8° and 24.1°;
preferably, the X-ray powder diffraction pattern of the crystal form of the fumarate represented by the diffraction angle of 2θ ± 0.2° at least comprises:
characteristic diffraction peaks shown at 5.5°, 7.0°, 9.2°, 10.6°, 13.9°, 18.4°, 21.8° and 24.1°;
more preferably, the X-ray powder diffraction pattern of the crystal form of the fumarate represented by the diffraction angle of 2θ ± 0.2° at least comprises:
characteristic diffraction peaks shown at 5.5°, 7.0°, 9.2°, 10.6°, 13.9°, 15.7°, 17.2°, 18.4°, 21.8° and 24.1°;
and particularly preferably, the crystal form of the fumarate has XRPD data substantially as shown in Table 1; and/or a XRPD pattern substantially as shown in FIG. 2.

9. The preparation method of the crystal form of the fumarate according to claim 8, comprising allowing 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to react with fumaric acid in a reaction solvent, the reaction solvent is an organic solvent or an aqueous solution of the organic solvent;
preferably, the organic solvent is a polar organic solvent;
further preferably, the organic solvent is selected from one or more of alcohol, ketone, halogenated alkane, ether and ester;
more preferably, the organic solvent is selected from one or more of C₁-C₅ linear or branched alkanol, acetone, THF, ethyl acetate, dichloromethane, and chloroform; particularly preferably, the organic solvent is selected from one or more of methanol, ethanol, propanol, isopropanol, acetone, dichloromethane and chloroform; more preferably, the organic solvent is selected from one or more of methanol, ethanol, dichloromethane and chloroform; most preferably, the organic solvent is ethanol, dichloromethane, or a combination of the both.

10. The method according to claim 9, wherein the method further comprises slowly adding fumaric acid dropwise into 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine for mixing; and/or
allowing 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine to react with fumaric acid at a temperature ranging from 20°C to a temperature at which the reaction solvent is refluxed; and/or
the method further comprises: dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine before reacting with fumaric acid.

11. The method according to claim 10, wherein the solvent used for dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine comprises a first solvent and a second solvent, wherein the first solvent is selected from one or more of a ketone, an ether, an ester and an alcohol, preferably selected from one or more alcohols, more preferably the first solvent is selected from one or more of C₁-C₅ linear and branched alkanols, particularly preferably selected from one or more of methanol, ethanol, propanol and isopropanol; the second solvent is dichloromethane, chloroform or a combination of the both;
preferably, the mixing volume ratio of the first solvent to the second solvent is 1:(0.1-10), preferably 1:(1-3), and most preferably 1:1.

12. The method according to any one of claims 9 to 11, wherein the method further comprises: after dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine, subjecting the resulting solution to filtration; and/or
the method further comprises: evaporating the solvent used for dissolving 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine before reacting with fumaric acid; and/or
the method further comprises: cooling the reaction system to a temperature of 10°C to 35°C, preferably cooling to a temperature of 20°C to 30°C after the reaction of 5-fluoro-4-(7'-fluoro-2'-methylspiro[cyclopentane-1,3'-indol]-5'-yl)-N-(5-(1-methylpiperidi n-4-yl)pyridin-2-yl)pyrimidine-2-amine with fumaric acid is completed.

13. A pharmaceutical composition, comprising a therapeutically effective amount of the salt according to any one of claims 1 to 6 or the crystal form of the fumarate according to any one of claims 7 to 8; preferably, the pharmaceutical composition also comprises a pharmaceutically acceptable adjuvant; more preferably, the pharmaceutically acceptable adjuvant includes at least one of a carrier, a diluent, and an excipient.

14. The pharmaceutical composition according to claim 13, wherein the pharmaceutical composition comprises one or more other biologically active substances;
preferably, the other biologically active substances are selected from one or more of an anticancer agent, an immunosuppressive agent and an anti-viral agent;
more preferably, the other biologically active substances are selected from one or more of an alkylating agent, an antineoplastic antimetabolite drug, a platinum complexing agent, an antibiotic antineoplastic drug, a naturally-derived antineoplastic drug, a hormonal antineoplastic drug, a VEGFR or EGFR inhibitor, an antineoplastic antibody drug, an mTOR inhibitor, and a drug for treating brain tumors; ;
particularly preferably,
the alkylating agent is selected from one or more of cyclophosphamide, ifosfamide, thiotepa, semustine, mechlorethamine hydrochloride, busulfan, chlorambucil, melphalan, nitrocaphane, formylmelphalan, carmustine, lomustine, altretamine, dibromomannitol, and temozolomide;
the antineoplastic antimetabolite drug is selected from one or more of cytarabine, fluorouracil, methotrexate, hydroxyurea, tegafur, Meisoindigo and mercaptopurine;
the platinum complexing agent is selected from one or more of cisplatin, carboplatin, and oxaliplatin;
the antibiotic antineoplastic drug is selected from one or more of actinomycin D, mitomycin, doxorubicin, pingyangmycin, epirubicin, pirarubicin, daunorubicin, and bleomycin;
the naturally-derived antineoplastic drug is selected from one or more of homoharringtonine and its derivatives, vincristine and its derivatives, hydroxycamptothecin and its derivatives, etoposide and its derivatives, vindesine and its derivatives, vinblastine and its derivatives, vinorelbine tartrate, taxol and its derivatives, colchicine and its derivatives, and elemene and its derivatives;
the hormonal antineoplastic drug is selected from one or more of aminoglutethimide, tamoxifen, dexamethasone, dutasteride, flutamide, gonadorelin, leuprolide acetate, and letrozole;
the VEGFR or EGFR inhibito is selected from one or more of sunitinib, sorafenib, imatinib, gefitinib, erlotinib, vandetanib, pazopanib, lapatinib, canertinib, afatinib, mubritinib, dasatinib, and neratinib;
the antineoplastic antibody drug is selected from one or more of trastuzumab, pertuzumab, rituximab, panitumumab, bevacizumab, ipilimumab, ofatumumab, and ramucirumab; the mTOR inhibitor comprises everolimus, sirolimus, and zotarolimus; and
the drug for treating brain tumors comprises temozolomide.

15. A kit, comprising the salt according to any one of claims 1 to 6 or the crystal form of the fumarate according to any one of claims 7 to 8, and one or more of other biologically active substances; preferably, thekit further comprises a pharmaceutically acceptable adjuvant; more preferably, the pharmaceutically acceptable adjuvant includes at least one of a carrier, a diluent, and an excipient;
preferably, the other biologically active substances are selected from one or more of an anticancer agent, an immunosuppressive agent and an anti-viral agent;
more preferably, the other biologically active substances are selected from one or more of an alkylating agent, an antineoplastic antimetabolite drug, a platinum complexing agent, an antibiotic antineoplastic drug, a naturally-derived antineoplastic drug, a hormonal antineoplastic drug, a VEGFR or EGFR inhibitor, an antineoplastic antibody drug, an mTOR inhibitor, and a drug for treating brain tumors;
particularly preferably,
the alkylating agent is selected from one or more of cyclophosphamide, ifosfamide, thiotepa, semustine, mechlorethamine hydrochloride, busulfan, chlorambucil, melphalan, nitrocaphane, formylmelphalan, carmustine, lomustine, altretamine, dibromomannitol, and temozolomide;
the antineoplastic antimetabolite drug is selected from one or more of cytarabine, fluorouracil, methotrexate, hydroxyurea, tegafur, Meisoindigo and mercaptopurine;
the platinum complexing agent is selected from one or more of cisplatin, carboplatin, and oxaliplatin;
the antibiotic antineoplastic drug is selected from one or more of actinomycin D, mitomycin, doxorubicin, pingyangmycin, epirubicin, pirarubicin, daunorubicin, and bleomycin;
the naturally-derived antineoplastic drug is selected from one or more of homoharringtonine and its derivatives, vincristine and its derivatives, hydroxycamptothecin and its derivatives, etoposide and its derivatives, vindesine and its derivatives, vinblastine and its derivatives, vinorelbine tartrate, taxol and its derivatives, colchicine and its derivatives, and elemene and its derivatives;
the hormonal antineoplastic drug is selected from one or more of aminoglutethimide, tamoxifen, dexamethasone, dutasteride, flutamide, gonadorelin, leuprolide acetate, and letrozole;
the VEGFR or EGFR inhibitor is selected from one or more of sunitinib, sorafenib, imatinib, gefitinib, erlotinib, vandetanib, pazopanib, lapatinib, canertinib, afatinib, mubritinib, dasatinib, and neratinib;
the antineoplastic antibody drug is selected from one or more of trastuzumab, pertuzumab, rituximab, panitumumab, bevacizumab, ipilimumab, ofatumumab, and ramucirumab; the mTOR inhibitor comprises everolimus, sirolimus, and zotarolimus; and
the drug for treating brain tumors comprises temozolomide.

16. The kit according to claim 15, wherein the other biologically active substance is temozolomide.

17. Use of the salt according to any one of claims 1 to 6, the crystal form of the fumarate according to any one of claims 7 to 8, the pharmaceutical composition according to claim 13-14, or the kit according to any one of claims 15 to 16 in the preparation of a medicament for the treatment of diseases responsive to the inhibition of a cyclin dependent kinase.

18. The use according to claim 17, wherein the diseases are selected from a brain tumor, a breast cancer, an urogenital cancer, a lung cancer, a gastrointestinal cancer, an epidermoid cancer, a melanoma, an ovarian cancer, a pancreatic cancer, a neuroblastoma, a head and neck cancer or a bladder cancer; a leukemia, a hyperplasia, a gastric cancer, a colon cancer, a laryngeal cancer, a lymphatic system cancer, a genitourinary tract cancer, a bone cancer, a prostate cancer, a small cell lung cancer, a glioma cancer, a colorectal cancer, a kidney cancer, an epithelial cancer, a liver cancer, an esophageal cancer, a hematopoietic system cancer, a lymphoma, a myeloma, a follicular thyroid cancer; a tumor of mesenchymal origin; a tumor of the central or peripheral nervous system; a seminoma; a teratocarcinoma; an osteosarcoma; xeroderma pigmentosum; a keratoacanthoma; a follicular thyroid cancer; Kaposi's sarcoma, chronic lymphocytic leukemia, mantle cell lymphoma, or large B-cell lymphoma;
preferably, the tumor of mesenchymal origin is a fibrosarcoma or a rhabdomyosarcoma; and the tumor of the central or peripheral nervous system is an astrocytoma, a neuroblastoma, a glioma, or a schwannomas.

19. The salt according to any one of claims 1 to 6, the crystal form of the fumarate according to any one of claims 7 to 8, the pharmaceutical composition according to claim 13 to 14, or the kit according to any one of claims 15 to 16, for use in the treatment of diseases responsive to the inhibition of a cyclin dependent kinase.

20. The salt, crystal form of fumarate, pharmaceutical composition or kit for the uses according to claim 19, wherein the diseases are selected from a brain tumor, a breast cancer, an urogenital cancer, a lung cancer, a gastrointestinal cancer, an epidermoid cancer, a melanoma, an ovarian cancer, a pancreatic cancer, a neuroblastoma, a head and neck cancer or a bladder cancer; a leukemia, a hyperplasia, a gastric cancer, a colon cancer, a laryngeal cancer, a lymphatic system cancer, a genitourinary tract cancer, a bone cancer, a prostate cancer, a small cell lung cancer, a glioma cancer, a colorectal cancer, a kidney cancer, an epithelial cancer, a liver cancer, an esophageal cancer, a hematopoietic system cancer, a lymphoma, a myeloma, a follicular thyroid cancer; a tumor of mesenchymal origin; a tumor of the central or peripheral nervous system; a seminoma; a teratocarcinoma; an osteosarcoma; xeroderma pigmentosum; a keratoacanthoma; a follicular thyroid cancer; Kaposi's sarcoma, chronic lymphocytic leukemia, mantle cell lymphoma, and large B-cell lymphoma;
preferably, the tumor of mesenchymal origin is a fibrosarcoma or a rhabdomyosarcoma; and the tumor of the central or peripheral nervous system is an astrocytoma, a neuroblastoma, a glioma, or a schwannomas.

21. A method for the treatment of diseases responsive to the inhibition of a cyclin dependent kinase, comprising administering a therapeutically effective amount of the salt according to any one of claims 1 to 6, the crystal form of the fumarate according to any one of claims 7 to 8, the pharmaceutical composition according to claim 13 to 14, or administering the kit according to any one of claims 15 to 16 to the individuals suffering from the said disease.

22. The method according to claim 21, wherein the diseases comprise a brain tumor, a breast cancer, an urogenital cancer, a lung cancer, a gastrointestinal cancer, an epidermoid cancer, a melanoma, an ovarian cancer, a pancreatic cancer, a neuroblastoma, a head and neck cancer or a bladder cancer; a leukemia, a hyperplasia, a gastric cancer, a colon cancer, a laryngeal cancer, a lymphatic system cancer, a genitourinary tract cancer, a bone cancer, a prostate cancer, a small cell lung cancer, a glioma cancer, a colorectal cancer, a kidney cancer, an epithelial cancer, a liver cancer, an esophageal cancer, a hematopoietic system cancer, a lymphoma, a myeloma, a follicular thyroid cancer; a tumor of mesenchymal origin; a tumor of the central or peripheral nervous system; a seminoma; a teratocarcinoma; an osteosarcoma; xeroderma pigmentosum; a keratoacanthoma; a follicular thyroid cancer; Kaposi's sarcoma, chronic lymphocytic leukemia, mantle cell lymphoma, and large B-cell lymphoma;
preferably, the tumor of mesenchymal origin is a fibrosarcoma or a rhabdomyosarcoma; and the tumor of the central or peripheral nervous system is an astrocytoma, a neuroblastoma, a glioma, or a schwannomas.
